# EUROPEAN PATENT SPECIFICATION

(11) **EP 3 658 566 B1**
(45) Date of publication and mention of the grant of the patent: **19.07.2023**
(21) Application number: 18750270.3
(22) Date of filing: 19.07.2018
(51) Int. Cl.: C07J 9/00, C07J 17/00, A61K 31/58, A61K 31/575, A61P 35/00, C07J 53/00, C07J 71/00

(54) **SIDE-CHAIN MODIFIED ERGOSTEROL AND STIGMASTEROL DERIVATIVES AS LIVER X RECEPTOR MODULATORS**
SEITENKETTENMODIFIZIERTE ERGOSTERIN- UND STIGMASTEROLDERIVATE ALS MODULATOREN DES LEBER-X-REZEPTORS
DÉRIVÉS D'ERGOSTÉROL ET DE STIGMASTÉROL MODIFIÉS PAR CHAÎNE LATÉRALE EN TANT QUE MODULATEURS DU RÉCEPTEUR HÉPATIQUE X

(30) Priority: 24.07.2017 EP 17182811
(43) Date of publication of application: 03.06.2020
(73) Proprietor: Ospedale San Raffaele S.r.l., 20132 Milano (IT)
(72) Inventor: RUSSO, Vincenzo, 20132 Milano (IT); MARINOZZI, Maura, 06134 Perugia (IT); CASTRO NAVAS, Francisco Fermin, 23710 Bailen (Jaén) (ES)
(74) Representative: Trupiano, Federica
(86) International application number: PCT/IB2018/055372
(87) International publication number: WO 2019/021122

(56) References cited:
- YU FLEGENTOV G ET AL: "22,23-Epoxides of Sitosterol and Related 7-Oxygenated [Delta]5-Sterols", RUSSIAN JOURNAL OF BIOORGANIC CHEMISTRY, KLUWER ACADEMIC PUBLISHERS-PLENUM PUBLISHERS, NE, vol. 31, no. 5, 1 September 2005 (2005-09-01), pages 475-481, XP019299976, ISSN: 1573-9163
- KANEKO E ET AL: "INDUCTION OF INTESTINAL ATP-BINDING CASSETTE TRANSPORTERS BY A PHYTOSTEROL-DERIVED LIVER X RECEPTOR AGONIST", JOURNAL OF BIOLOGICAL CHEMISTRY, AMERICAN SOCIETY FOR BIOCHEMISTRY AND MOLECULAR BIOLOGY, US, vol. 278, no. 38, 1 January 2003 (2003-01-01), pages 36091-36098, XP001199518, ISSN: 0021-9258, DOI: 10.1074/JBC.M304153200
- MISHARIN ET AL: "Synthesis and cytotoxicity evaluation of 22,23-oxygenated stigmastane derivatives", BIOORGANIC & MEDICINAL CHEMISTRY, PERGAMON, GB, vol. 16, no. 3, 22 October 2007 (2007-10-22), pages 1460-1473, XP022453115, ISSN: 0968-0896, DOI: 10.1016/J.BMC.2007.10.056
- KEN-ICHI MORITA ET AL: "Syntheses of 23-Ketositosterol and Its Derivatives", BULLETIN OF THE CHEMICAL SOCIETY OF JAPAN, vol. 36, no. 10, 1 October 1963 (1963-10-01), pages 1332-1337, XP055512640, JP ISSN: 0009-2673, DOI: 10.1246/bcsj.36.1332
- NIGEL BOSWORTH ET AL: "Unsaturated steroids. Part 2. A novel route to anthrasteroids: X-ray crystal structure of 1(10 ? 6)abeo-cholesta-5,7,9-trien-3-yl p-bromobenzoate", JOURNAL OF THE CHEMICAL SOCIETY, PERKIN TRANSACTIONS 1, no. 8, 1 January 1977 (1977-01-01), pages 805-809, XP055512634, ISSN: 0300-922X, DOI: 10.1039/p19770000805
- PATRICK KOCH ET AL: "Identification de stérols à chaîne latérale oxygénée dans une éponge de l'espèce Hyrtios", HELVETICA CHIMICA ACTA, vol. 66, no. 8, 14 December 1983 (1983-12-14), pages 2431-2436, XP055512604, CH ISSN: 0018-019X, DOI: 10.1002/hlca.19830660806
- MASAO TADA ET AL: "Synthesis of 22,23-epoxyvitamin D2(22,23-epoxyergocalciferol)", JOURNAL OF THE CHEMICAL SOCIETY, PERKIN TRANSACTIONS 1, 1 January 1979 (1979-01-01), pages 1858-1861, XP055512592, ISSN: 0300-922X, DOI: 10.1039/p19790001858
- JOHN T. GROVES ET AL: "Regioselective oxidation catalysis in synthetic phospholipid vesicles. Membrane-spanning steroidal metalloporphyrins", JOURNAL OF THE AMERICAN CHEMICAL SOCIETY, vol. 111, no. 8, 1 April 1989 (1989-04-01) , pages 2900-2909, XP055512368, US ISSN: 0002-7863, DOI: 10.1021/ja00190a026
- FUMIHIRO KONDO ET AL: "Facile retro-cycloaddition of adducts derived from steroidal 5,7-diene and 4-phenyl-1,2,4-triazoline-3,5-dione by diisobutylaluminium hydride", JOURNAL OF THE CHEMICAL SOCIETY, PERKIN TRANSACTIONS 1, vol. 1, no. 21, 1 January 1995 (1995-01-01), pages 2679-2680, XP055512361, ISSN: 0300-922X, DOI: 10.1039/p19950002679
- MAURA MARINOZZI ET AL: "Side-Chain Modified Ergosterol and Stigmasterol Derivatives as Liver X Receptor Agonists", JOURNAL OF MEDICINAL CHEMISTRY, vol. 60, no. 15, 25 July 2017 (2017-07-25) , pages 6548-6562, XP055511605, ISSN: 0022-2623, DOI: 10.1021/acs.jmedchem.7b00091

## Description

### Summary of the invention

The present invention relates to Liver X Receptor (LXR) modulators, for use in therapy, more particularly for use in the treatment of diseases associated with LXR, such as cancer, inflammation, metabolic and autoimmune diseases.

### Technical Background

Oxysterols are 27-carbon intermediates or end-products of cholesterol metabolism, structurally characterized by the presence of oxygenated functions such as hydroxy, keto, hydroperoxy, epoxy and carboxy moieties. They are produced *in vivo* through both enzymatic- and non-enzymatic (auto-oxidation) processes.^{1,2} Specific enzymes of the cytochrome P450 (CYP) family preferentially oxidize the cholesterol side chain (7α-hydroxycholesterol (**1a**), 24(*S*)-hydroxycholesterol (**2**), 22(*R*)-hydroxycholesterol (22*R*-HC, **3**), and 24(*S*),25-epoxycholesterol (**4**) are examples of oxysterols generated by CYPs, see below), whereas the double bond of the cholesterol B-ring represents a privileged target for free-radical-involving reactions. Thus, 7-ketocholesterol (**5**), 7β-hydroxycholesterol (**1b**), 5α,6α- and 5β,6β-epoxycholesterols (**6a,b**) constitute the main non-enzymatically produced oxysterols (see below).^{1,2}

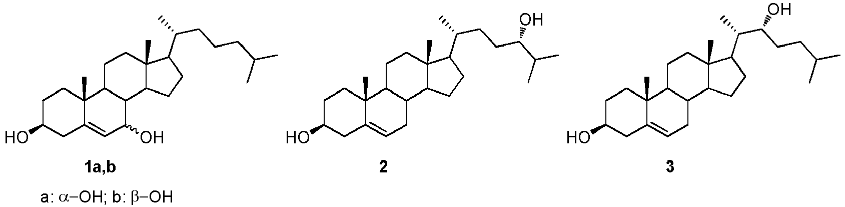

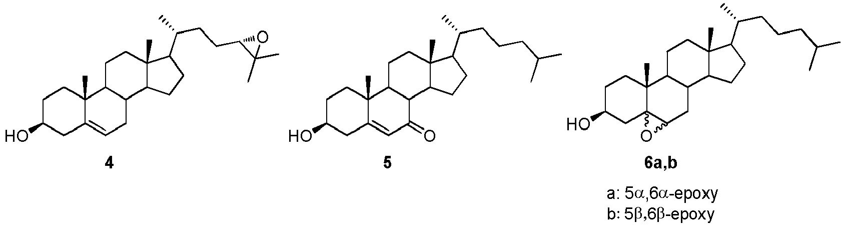

A broader definition for the class of oxysterols is not limited to cholesterol oxidation products, but includes also steroidal oxygenated derivatives that humans can assimilate by diet, either as primary constituents (plants and shellfish sterols) or as storage and cooking-derived components.¹

The past two decades have evidenced an exponential increase in the number of studies on the physiological roles of mammalian oxysterols, as well as on their contribution to the pathogenesis of different diseases.^{3,4,5,6} The major breakthrough was the identification of a specific subset of oxysterols (**2-4**)^{7,8} as endogenous ligands of Liver X Receptor α and β (LXRs).^{9,10,11,12,13} Thus, given the action of LXRs (α and β isoforms) as whole-body cholesterol sensors and key regulators of lipogenesis, oxysterols have the potential to assume a key role in the modulation of lipid metabolism and glucose homeostasis.

LXRs and their ligands can also suppress inflammatory responses, either by activating the genes that encode anti-inflammatory proteins or by suppressing the genes that are under the control of proinflammatory transcription factors.^{4,14}

However, the functions of oxysterols are not limited to their LXR binding,¹⁵ but they significantly interact with other cellular proteins, giving rise to different effects. Examples of proteins affected by oxysterols are: a) insulin-induced gene (INSIG) proteins, regulating the function of sterol response element binding protein (SREBP);¹⁶ b) Niemann-Pick C1 (NPC1) and oxysterol-binding protein family (OSBP/ORP), involved in cholesterol metabolism;¹⁷ and c) Smoothened oncoprotein, interfering with the Hedgehog signalling.¹⁸

So far the oxysterol medicinal chemistry has been mainly focused on the identification of LXR modulators, although the number of the studied natural and synthetic oxysterol derivatives is only marginal when compared to that of the non-steroidal ligands.^{19,20} The first series of synthetic steroidal ligands allowed the identification of the minimal pharmacophore for LXRα, i.e. a sterol with a hydrogen bond acceptor at C-24.²¹ The most potent derivative of this series, namely cholenic acid dimethylamide 7, was an efficacious LXRα agonist,²¹ able to promote a gene-selective modulation (see below).²² 5α,6α-Epoxycholesterol (**6a**), identified in processed food, was shown to be a LXR modulator with cell and gene-context-dependent activities,²³ whereas the two 5β-cholane derivatives 3α,6α,24-trihydroxy-24,24-di(trifluoromethyl)-5β-cholane (ATI-829, **8**)²⁴ and 3α,6α,24-trihydroxy-22-en-24,24-di(trifluoromethyl)-5β-cholane (ATI-111, **9**),²⁵ whose design was inspired by the structure of the potent non-steroidal agonist *N-*(2,2,2-trifluoroethyl)-*N-*[4-[2,2,2-trifluoro-1-hydroxy-1-(trifluoromethyl)ethyl]phenyl]benzene sulfonamide (T0901317),²⁶ demonstrated antiatherosclerotic effects.^{24,25} In view of the well-known effect of phytosterols in reducing blood cholesterol,²⁷ and considering the fact that the treatment of intestinal cells with these compounds was found to increase the expression of LXR target genes,²⁸ Kaneko *et al.²⁹* studied the LXR activity of a series of phytosterols, including natural and semi-synthetic derivatives. They identified (22*E*)-ergost-22-ene-1α,3β-diol (YT-32, **10**)²⁹ as a potent and non-isoform selective LXR agonist, able to selectively induce the expression of ABC transporter genes in the intestine. Interestingly, the oral administration of **10** resulted in the inhibition of the intestinal cholesterol adsorption without increasing plasma triglyceride levels, in contrast to what observed with non-steroidal ligands.^{19,30}

The study of phytosterols as LXR agonists is limited to the mentioned compound **10,** to the plant hormone 28-homobrassinolide (**11**),³¹ and to 24(*S*)-saringosterol (**12**), a minor component isolated from marine seaweeds which showed to act as a selective LXRβ agonist.³²

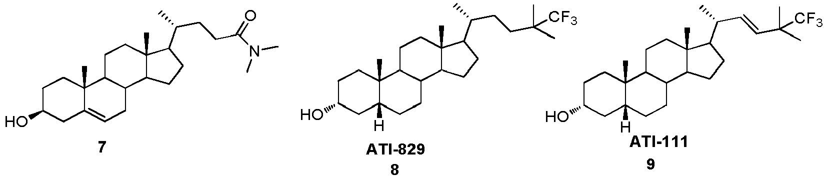

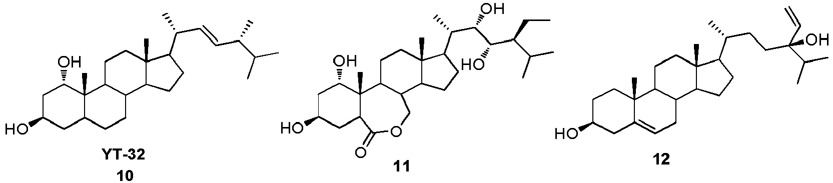

Indeed, the medicinal chemistry of Liver X Receptor (LXR) modulators has been so far mainly focused on non-steroidal compounds. Despite some compounds endowed with high potency have been discovered, these are characterized by a low or null gene-selectivity, thus actually preventing their clinical applications. Indeed, the non-steroidal compounds T0901317 and GW3965 having the following formulas 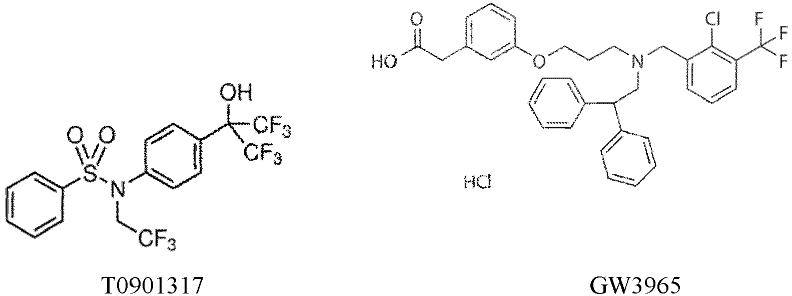 induce lipogenic effects by increasing liver and circulating triglyceride levels. The lipogenic effects have precluded further clinical development of these compounds.

To date, few steroidal modulator so far reported have shown cell and gene-context-dependent activities. There is therefore a need for steroid-based LXR modulators which are endowed with a better pharmacokinetic properties and lower toxicity with respect of LXR agonists of the prior art.

Russ. J. Bioorg. Chem. Vol. 31(5), 475-481, 2005, discloses the synthesis of (22R,23R)- and (22S,23S)-22,23-epoxystigmast-5-ene-3β-ol but no information is given concerning the possible activity of said compound. The document generally reports that oxysterols control the activity of LXR and presumes that some derivatives could be more stable and effective in the regulation of liver lipid metabolism.

### Objects of the invention

It is an object of the invention to provide steroid-based compounds which are LXR modulators, particularly agonists., for use in therapy.

It is a further scope of the invention to provide said steroid-based LXR modulators, especially agonists, for use in therapy, especially in the treatment of diseases associated with LXR, such as cancer, inflammation, metabolic and autoimmune diseases.

It is further scope of the invention to provide pharmaceutical compositions comprising said steroid-based LXR modulators for use in therapy.

### Definitions

In the present application the following abbreviation will be used:
ABCA1, ATP-binding cassette transporter A1;
CYP, cytochrome;
DMF, dimethyl formamide;
DMSO, dimethyl sulfoxide;
FASN, fatty acid synthase;
FXR, farnesoid X receptor;
hLXR, human liver X receptor;
INSIG, insulin-induced gene; LPS, lipopolysaccharides;
LXR, liver X receptor;
MCP-1, monocyte chemoattractant protein-1;
mCPBA, m-chloroperoxybenzoic acid;
mpc, medium pressure chromatography;
NPC1, Niemann-Pick C1;
ORP, OSBP-related protein;
OSBP, oxysterol-binding protein;
PCR, polymerase chain reaction;
PMA, phorbol 12-myristate 13-acetate;
PPARγ, peroxisome proliferator-activated receptor γ;
PPTS, pyridinum p-toluenesulfonate;
PXR, pregnane X receptor;
qPCR, quantitative polymerase chain reaction;
RNA, ribonucleic acid; RXR, retinoid X receptor;
SCD1, stearoyl-CoA desaturase 1;
SD, standard deviation;
SREBP, sterol response element binding protein;
TLR, toll-like receptor;
TNF, tumor necrosis factor.

### Brief description of the Figures

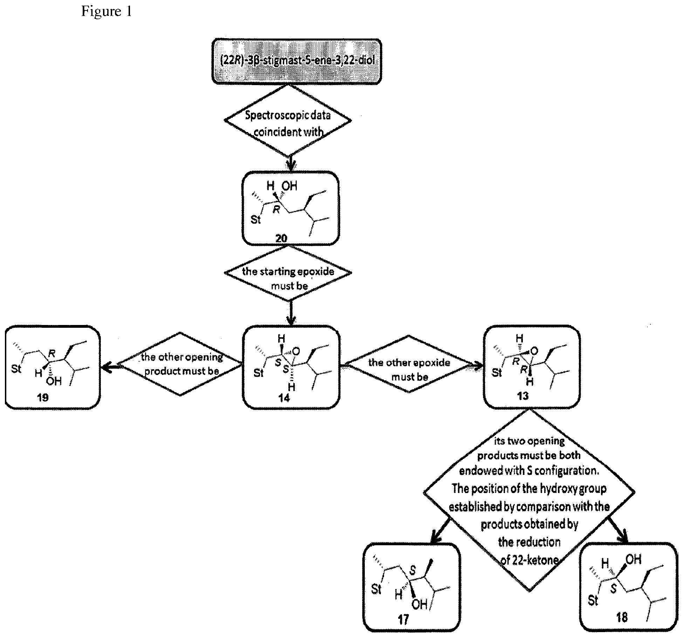

Figure 1 shows the flowchart for the structural assignment of the stigmastane derivatives.

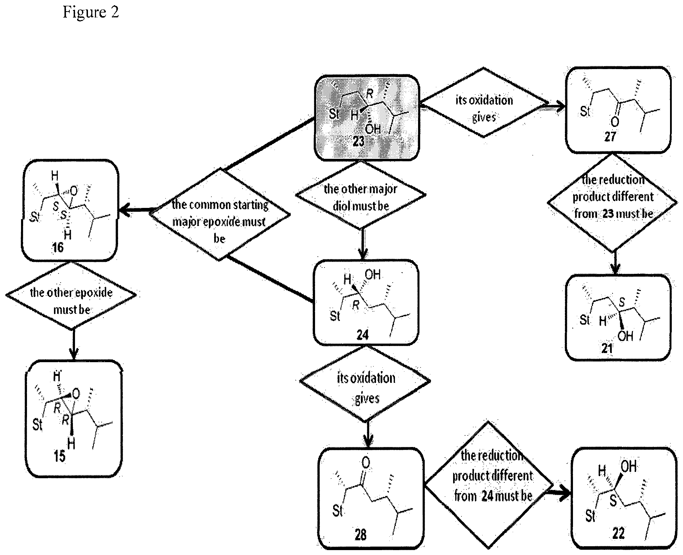

Figure 2 shows the flowchart for the structural assignment of the ergostane derivatives.

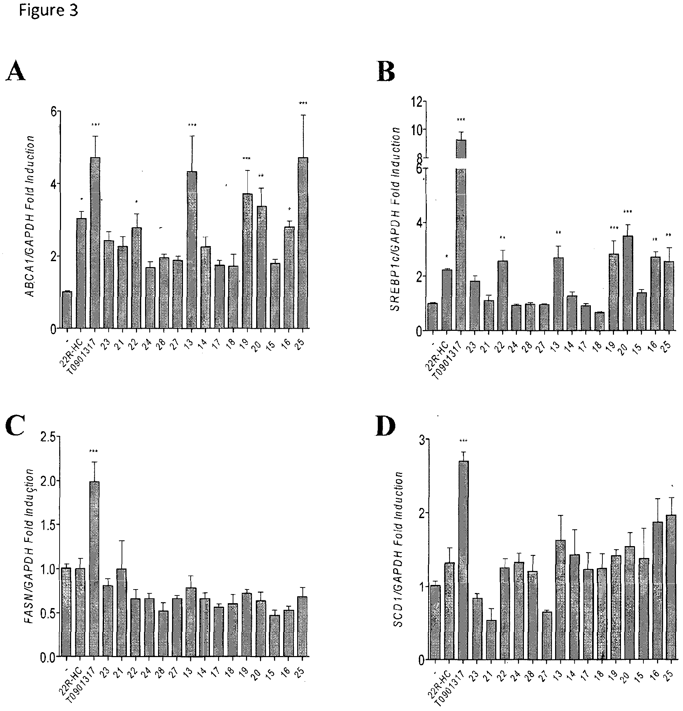

Figure 3 shows the regulation of ABCA1 (A), SREBP1c (B), FASN (C), and SCD1 (D) genes by the title compounds assessed by qPCR. U937 cells differentiated with PMA for 72 hours were treated with T0901317 (10 µM), 22R-HC (3) or with the tested compound (10 µM). The results show mean ± SD of three biological samples. (n = 3/group). *p < 0.05, **p < 0.01, ***p < 0.001.

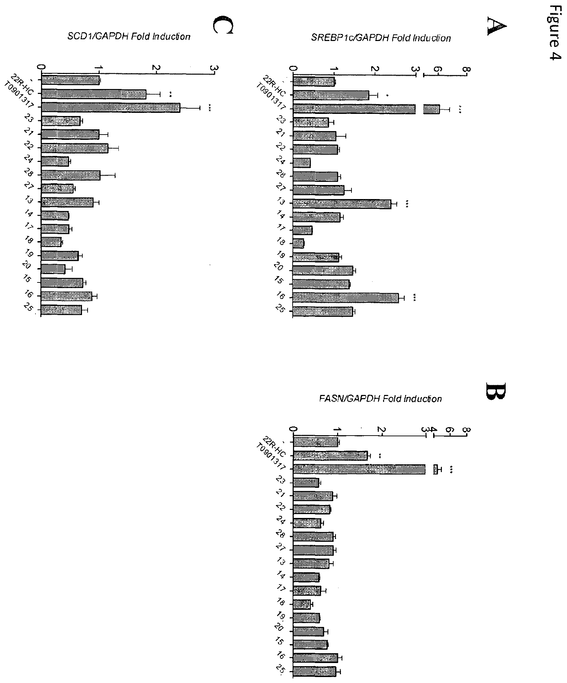

Figure 4 shows the regulation of SREBP1c (A), FASN (B), and SCD1 (C) genes by the title compounds assessed by qPCR. HepG2 cells were treated with T0901317 (10 µM), 22R-HC (3) (10 µM) or with the tested compound (10 µM). The results show mean ± SD of three biological samples. (n = 3/group). *p < 0.05, **p < 0.01, ***p < 0.001.

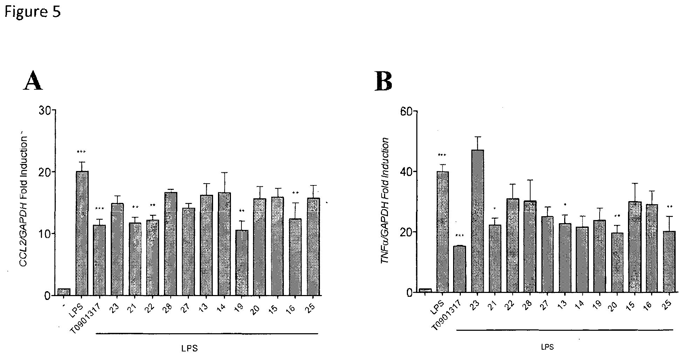

Figure 5 shows the regulation of CCL2 (A) and TNFα (B) genes by the title compounds assessed by qPCR. U937 cells differentiated with PMA for 72 hours were treated with LPS (100 ng/ml) in combination with T0901317 (10 µM) or with the title compounds (10 µM). The results show mean ± SD of three biological samples. (n = 3/group). *p < 0.05, **p < 0.01, ***p < 0.001.

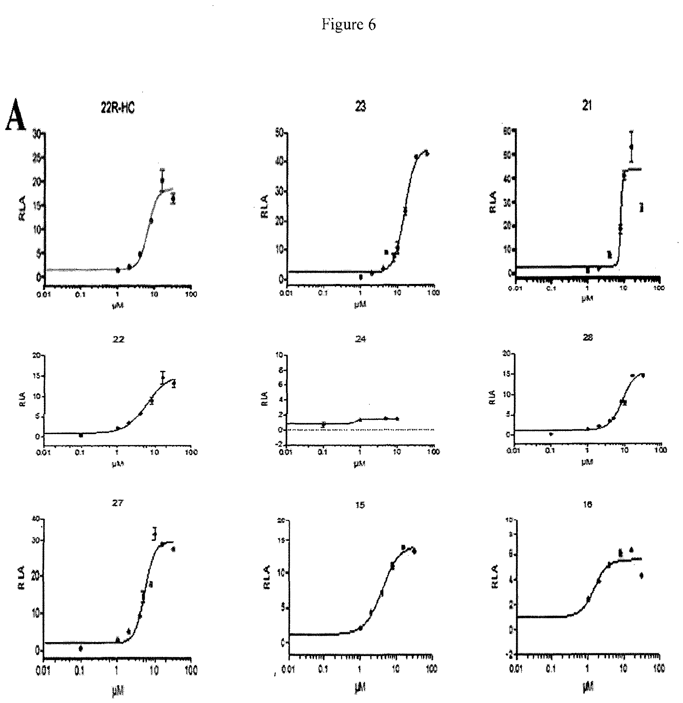

Figure 6 shows ergosterol derivatives activate LXRα. Human embryonic kidney 293 cells were co-transfected with the GAL4-hLXRα expression plasmid together with GAL4 responsive element pMHC100X4-TK-luc and Renilla plasmids. After 24 hours cells were incubated with different concentrations of synthetic agonists derived from the ergosterol and assayed for luciferase activity. Luciferase activity is expressed as fold induction of the compound over the vehicle. The values represent mean ±S.D. RLA, relative luciferase activity

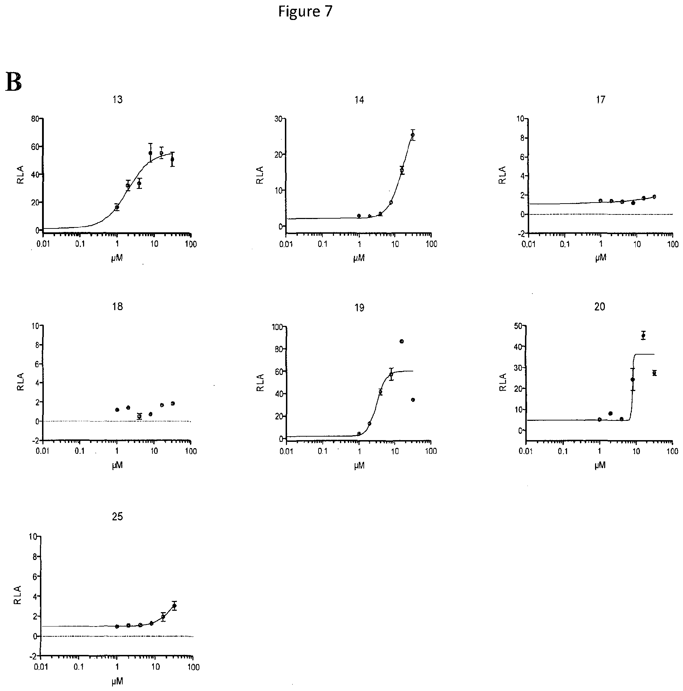

Figure 7 shows stigmasterol derivatives activate LXRα. Human embryonic kidney 293 cells were co-transfected with the GAL4-hLXRα expression plasmid together with GAL4 responsive element pMHC100X4-TK-luc and Renilla plasmids. After 24 hours cells were incubated with different concentrations of synthetic agonists derived from the stigmasterol and assayed for luciferase activity. Luciferase activity is expressed as fold induction of the compound over the vehicle. The values represent mean ±S.D. RLA, relative luciferase activity.

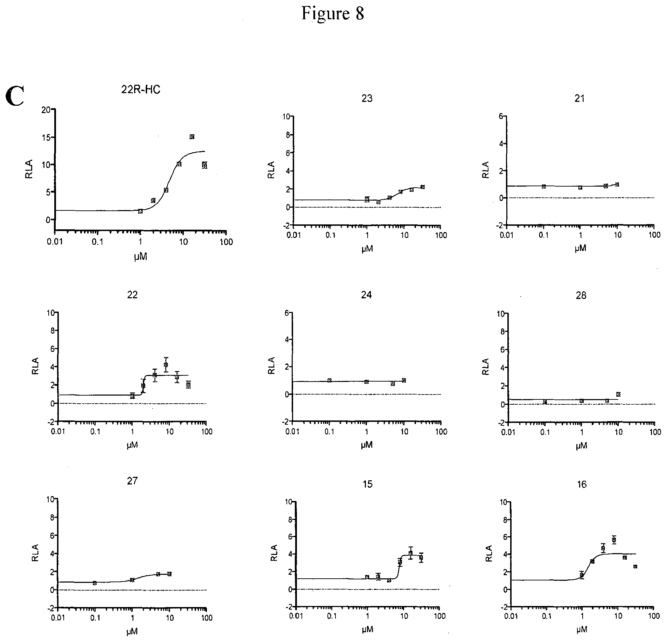

Figure 8 shows ergosterol derivatives activate LXRβ. Human embryonic kidney 293 cells were co-transfected with the GAL4-hLXRβ expression plasmid together with GAL4 responsive element pMHC100X4-TK-luc and Renilla plasmids. After 24 hours cells were incubated with different concentrations of synthetic agonists derived from the ergosterol and assayed for luciferase activity. Luciferase activity is expressed as fold induction of the compound over the vehicle. The values represent mean ±S.D. RLA, relative luciferase activity.

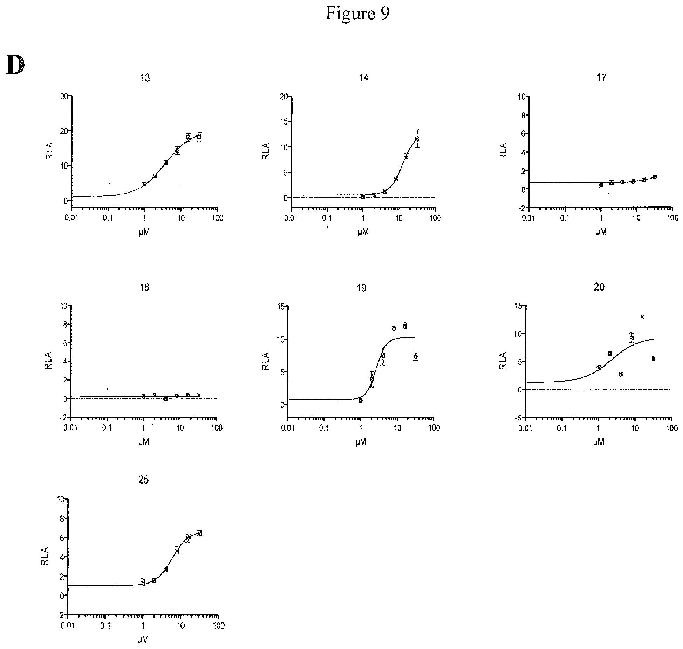

Figure 9 Shows stigmasterol derivatives activate LXRβ. Human embryonic kidney 293 cells were co-transfected with the GAL4-hLXRβ expression plasmid together with GAL4 responsive element pMHC100X4-TK-luc and Renilla plasmids. After 24 hours cells were incubated with different concentrations of synthetic agonists derived from the stigmasterol and assayed for luciferase activity. Luciferase activity is expressed as fold induction of the compound over the vehicle. The values represent mean ±S.D. RLA, relative luciferase activity.

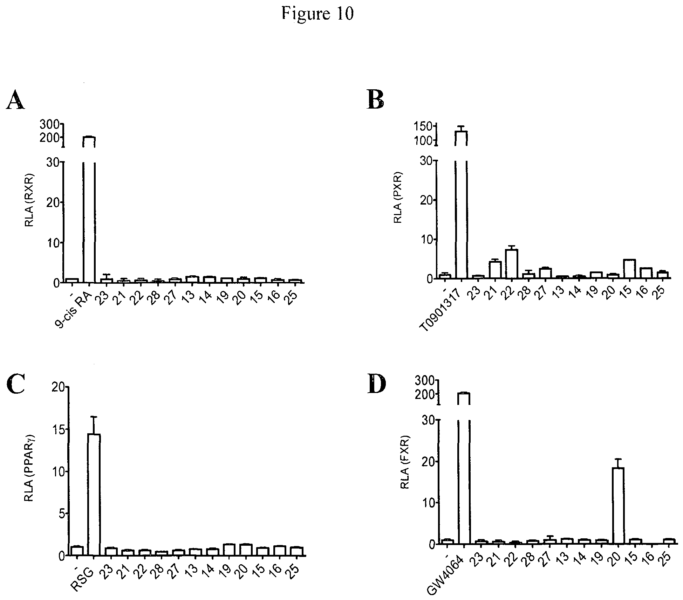

Figure 10 shows analysis of RXR, PPARγ, PXR and FXR nuclear receptors' activation by the ergosterol and stigmasterol derivatives. Human embryonic kidney 293 cells were co-transfected with GAL4-hRXR (A), GAL4-hPPARy (B), GAL4-hPXR (C) and GAL4-hFXR (D) expression plasmids together with GAL4 responsive element pMHC100X4-TK-luc and Renilla plasmids. Cells were incubated with vehicle (DMSO), PFMs at the concentration of 10 µM, and the nuclear receptor specific ligands 9cis retinoic acid (100 nM) (A), Rosiglitazone (RSG 100 nM) (B), T0901317 (100 nM) (C) and GW4064 (100 nM) (D) for six hours. The values represent mean ±S.D. RLA, relative luciferase activity.

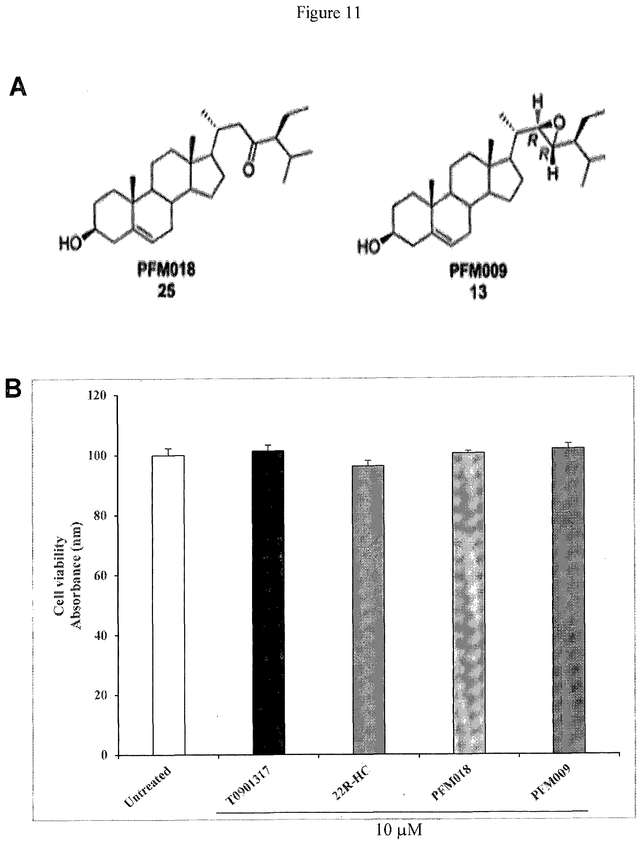

Figure 11 shows the in vitro effects of PFM009 (13) and PFM018 (25) on LLC tumor cells.

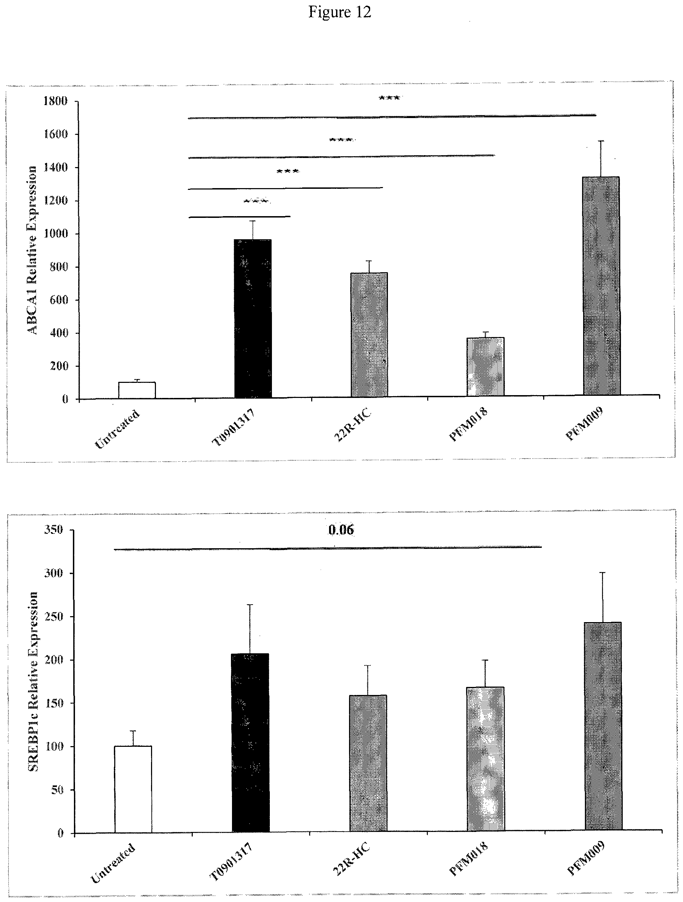

Figure 12 shows the expression of the LXR target gene Abca1 in LLC tumor cells induced by PFM009 (13) and PFM018 (25).

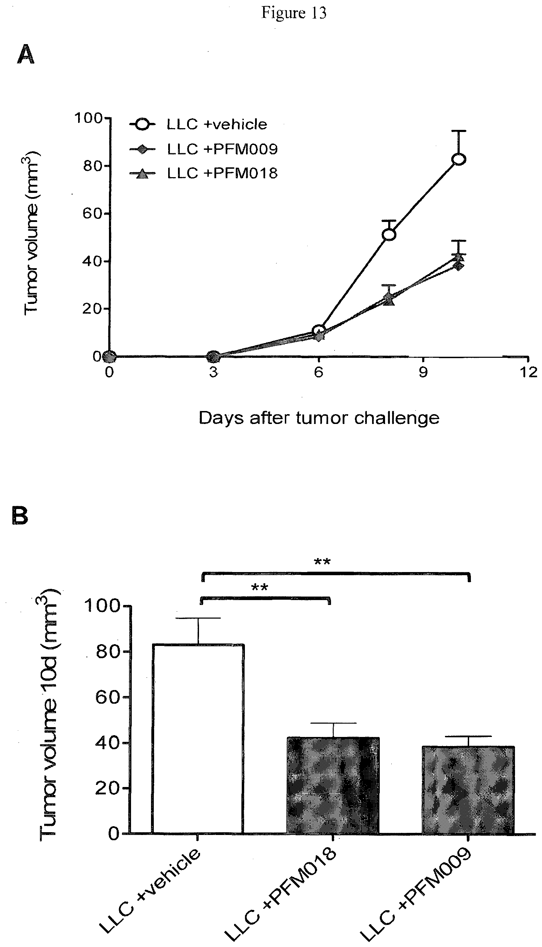

Figure 13 shows the controlling effects of LLC tumor growth in mice were treated with PFM009 (13) and PFM018 (25).

### Description of the invention

According to one of its aspects, the invention relates to compounds of formula (I) 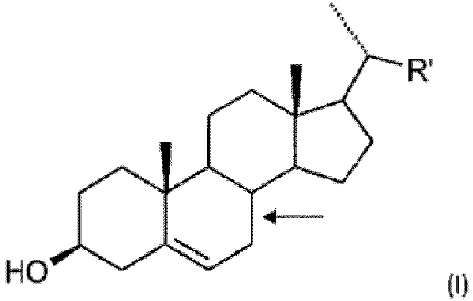 wherein
the bond indicated by the arrow is a single or a double bond; and
R' is the following group: 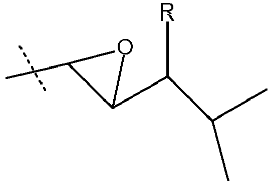 wherein
   - the dotted line indicates the bond by which said group is linked to the remaining part of the molecule;
   - R is a linear or branched, saturated C₁-C₆-alkyl, preferably C₁-C₄-alkyl; and
for use in therapy.

According to the present invention, the linear or branched, saturated C₁-C₆-alkyl, preferably C₁-C₄-alkyl, is more preferably a methyl or an ethyl group.

According to the present invention, compound (I) is selected from the following formulas

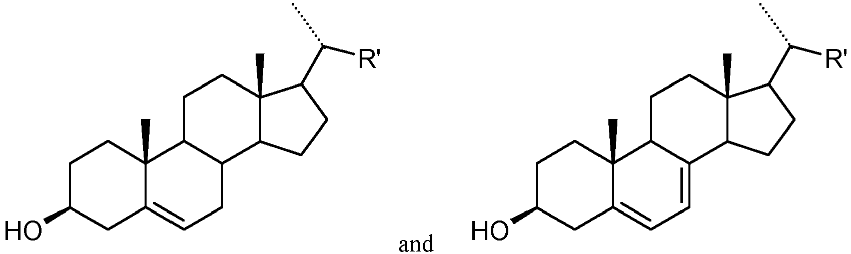

Particularly preferred compounds are those having the following formulas 13 to 16:

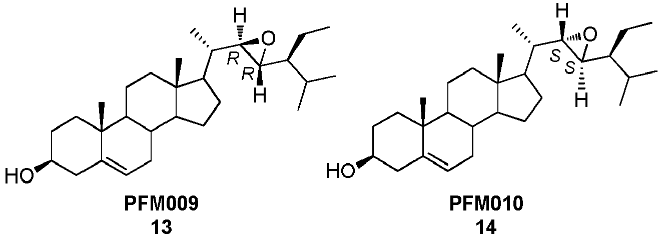

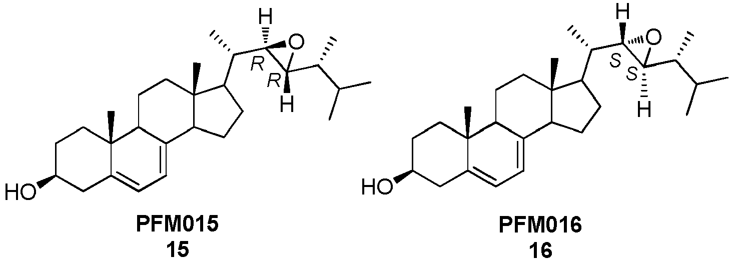

Particularly preferred compounds is compound 13.

The compounds of the invention are useful as LXR modulators, especially as LXRα and/or LXRβ agonists, especially compound 13 is a non-selective LXR agonist.

The compounds of the present invention may be prepared according to methods known in the art.

For instance, for the above compounds, epoxides 13-16, may be obtained by oxidizing the double bond between positions C-22 and C-23 of stigmasterol and ergosterol.

(22E)-3α,5α-cyclo-6β-methoxystigmast-22-ene (29), obtained in two steps from stigmasterol, as already reported, 34 represented the starting material for the preparation of the stigmastane derivatives (Scheme 1). The epoxidation reaction of 29 resulted in the formation of the two diastereoisomeric epoxides 30 and 31, which were separated by chromatography in 30 and 18% yield, respectively.35 The recovery of the 3β-hydroxy-5,6-ene moiety was performed by the known two-step procedure,35 consisting first in the treatment with glacial acetic acid, followed by the alkaline hydrolysis in the presence of hydroalcoholic potassium carbonate solution. Thus, starting from 30 and 31, the desired (22R,23R)-22,23-epoxystigmast-5-ene-3β-ol (13) and its (22S,23S)-isomer 14, respectively were obtained. 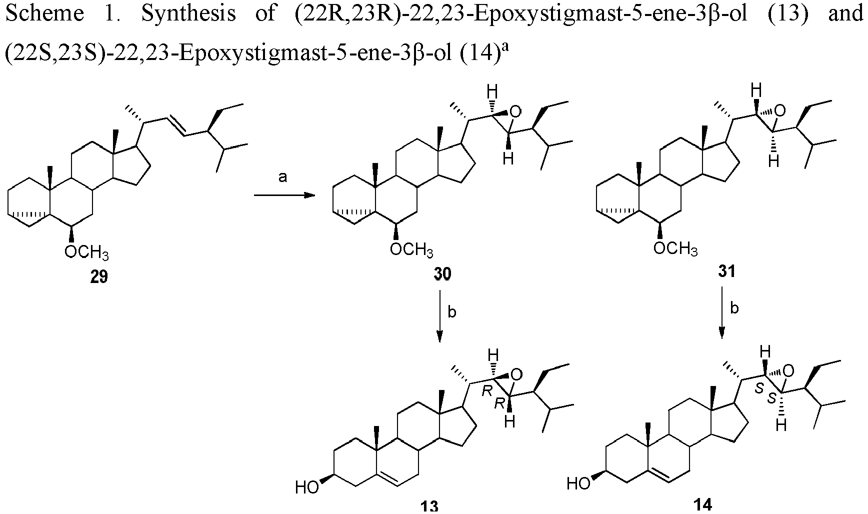 ^{a}Reagents and conditions: (a) *i.* m-CPBA, NaHCO₃, CH₂Cl₂, reflux, 2 h; *ii.* mpc; (b) i. glacial AcOH, reflux, 5h; *ii.* K₂CO₃, MeOH/H₂O, reflux, 3 h.

The LiAlH4-promoted reductive opening of the oxirane ring of 30 (Scheme 2) gave the inseparable mixture of the corresponding 23S- and 22S-hydroxy derivatives 32 + 33, which was first treated with glacial acetic acid and then in basic conditions to afford, after medium pressure chromatography (mpc), pure samples of (23S)-3β-stigmast-5-ene-3,23-diol (17) and (22S)-3β-stigmast-5-ene-3,22-diol (18), however, neither this compound nor its indication for any therapeutic use forms part of the present invention. 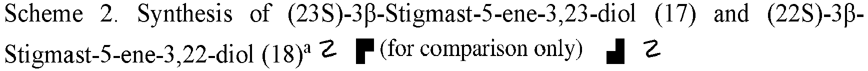 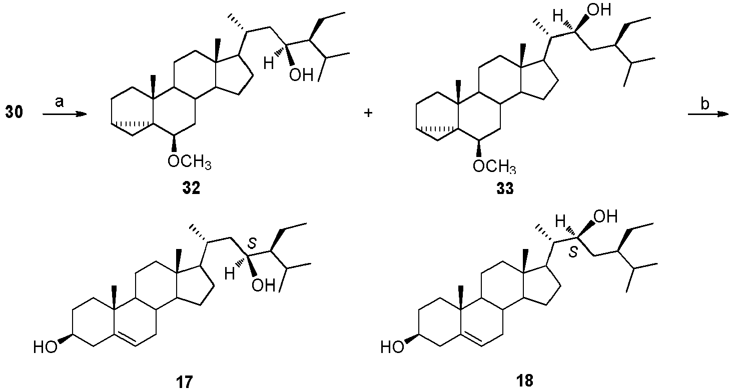 ^{a}Reagents and conditions: (a) LiAlH₄, THF, reflux, 36 h; (b) *i.* glacial AcOH, reflux, 6 h; *ii.* 2M KOH, MeOH, reflux, 3h; *iii.* mpc.

Similarly, the reductive opening of the epoxide 31 gave the inseparable mixture of the corresponding 23R- and 22R-hydroxy derivatives 34 + 35 (Scheme 3). In this case, the chromatographic separation of the two components of the mixture was only possible as 3β-acetate form. Thus, the mixture 34 + 35 was heated in glacial acetic acid and the crude submitted to mpc to achieve the two pure isomers 36 and 37. Their final alkaline hydrolysis gave the desired (23R)-3β-stigmast-5-ene-3,23-diol (19) and (22R)-3β-stigmast-5-ene-3,22-diol (20), respectively, thus completing the series of stigmastanediols, however, neither compound (19) nor compound (20) nor any therapeutic indications thereof form part of the present invention. 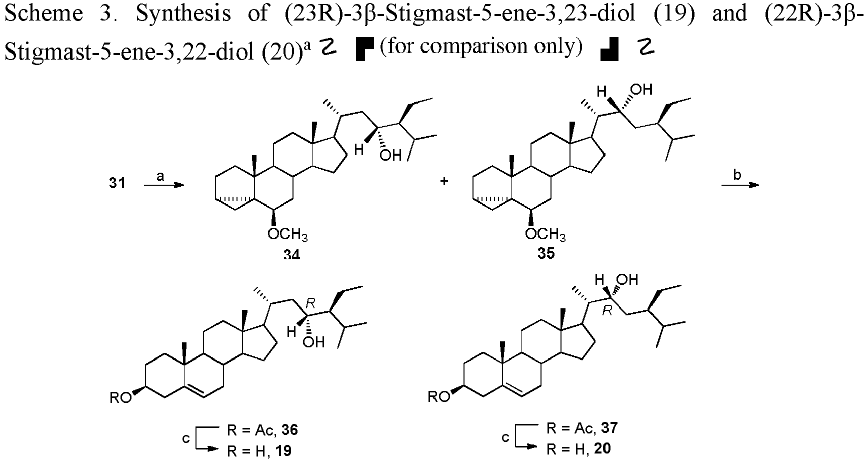 ^{a}Reagents and conditions: (a) LiAlH₄, THF, reflux, 36 h; (b) *i.* glacial AcOH, reflux, 6h; *ii.* mpc; (c) 2M KOH, MeOH, reflux, 3h.

Swern oxidation of (23R)-3β-acetoxystigmast-5-ene-23-ol (36) afforded the corresponding 23-keto derivative 38 (Scheme 4), which under basic hydrolysis gave the desired 3β-hydroxystigmast-5-ene-23-one (25), , however, neither this compound nor its indication for any therapeutic use forms part of the present invention. 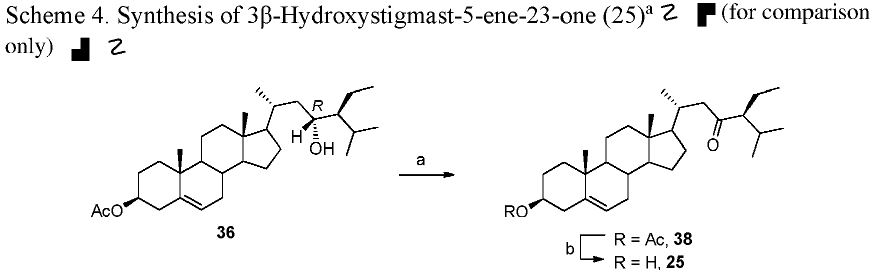 ^{a}Reagents and conditions: (a) (COCl)₂, DMSO, CH₂Cl₂, -78°C, 2h, then Et₃N, r.t.; (b) 2M KOH, acetone, reflux, 3h.

Analogously, (22R)-3β-acetoxystigmast-5-ene-22-ol (37) was converted into the desired 3β-hydroxystigmast-5-ene-22-one (26) (Scheme 5), however, neither this compound nor its indication for any therapeutic use forms part of the present invention. 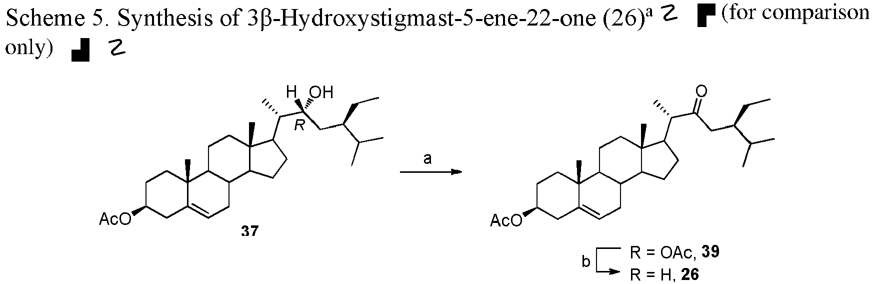 ^{a}Reagents and conditions: (a) (COCl)₂, DMSO, CH₂Cl₂, -78°C, 2h, then Et₃N, r.t.; (b) 2M KOH, acetone, reflux, 3h.

3β-Acetoxy cycloadduct 40, obtained by Diels-Alder cycloaddition between ergosterol-3β-acetate and 4-phenyl-1,2,4-triazoline-3,5-dione,36 constituted the starting material for the synthesis of the ergostane derivatives: Its epoxidation reaction with mCPBA gave access to the inseparable mixture of the two diastereoisomeric epoxides 41a.36 In an analogous manner the corresponding mixture of 3β-tetrahydropyranyl-protected epoxides 41b was also prepared starting from 3β-tetrahydropyranyloxy cycloadduct.37 The treatment of the mixture 41a with anhydrous potassium carbonate resulted in the retro 1,4-cycloaddition reaction, affording, after mpc, the two single isomers 42 and 43 (Scheme 6). The minor, less polar component 42, whose absolute configuration was assigned as 22R,23R (vedi infra), was submitted to alkaline hydrolysis to furnish (22R,23R)-22,23-epoxyergosta-5,7-diene-3β-ol (15). The same procedure starting from the major, more polar epoxide 43 gave the corresponding (22S,23S)-22,23-epoxyergosta-5,7-diene-3β-ol (16). 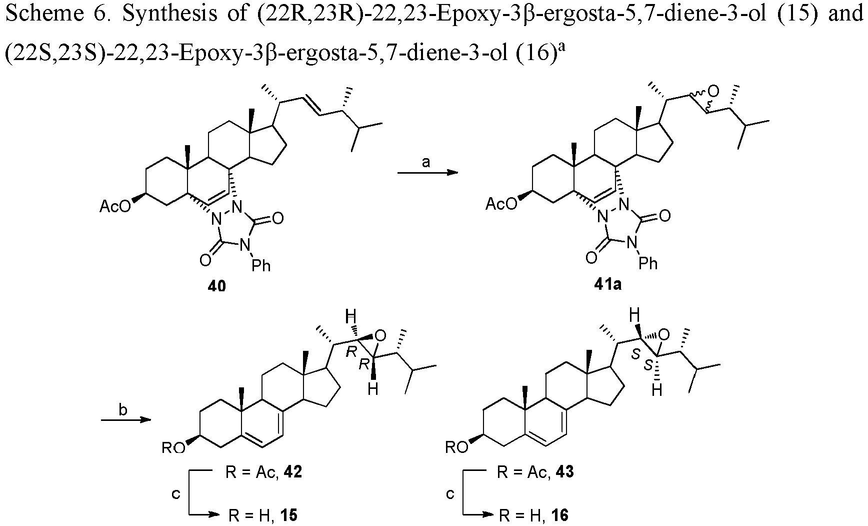 ^{a}Reagents and conditions: (a) *m*CPBA, CH₂Cl₂, r.t., 5h; (b) K₂CO₃, DMF, reflux, 6h; (c) 2M KOH, EtOH, reflux, 15 min.

The reductive opening of the epoxide mixture 41b gave, after separation by mpc, three different fractions, constituted by (23R)-3β-tetrahydropyranyloxyergost-5,7-diene-23-ol (44), the inseparable mixture of (23S)- and (22S)-3β-tetrahydropyranyl-protected diols (45 + 46), and (22R)-3β-tetrahydropyranyloxyergost-5,7-diene-22-ol (47) (Scheme 7). The deprotection of the 3β-hydroxy group of 44 by pyridinium p-toluenesulfonate (PPTS)38 provided the desired (23R)-3β-ergost-5,7-diene-3,23-diol (23), however, none of these products nor any therapeutic indication thereof forms part of the present invention. 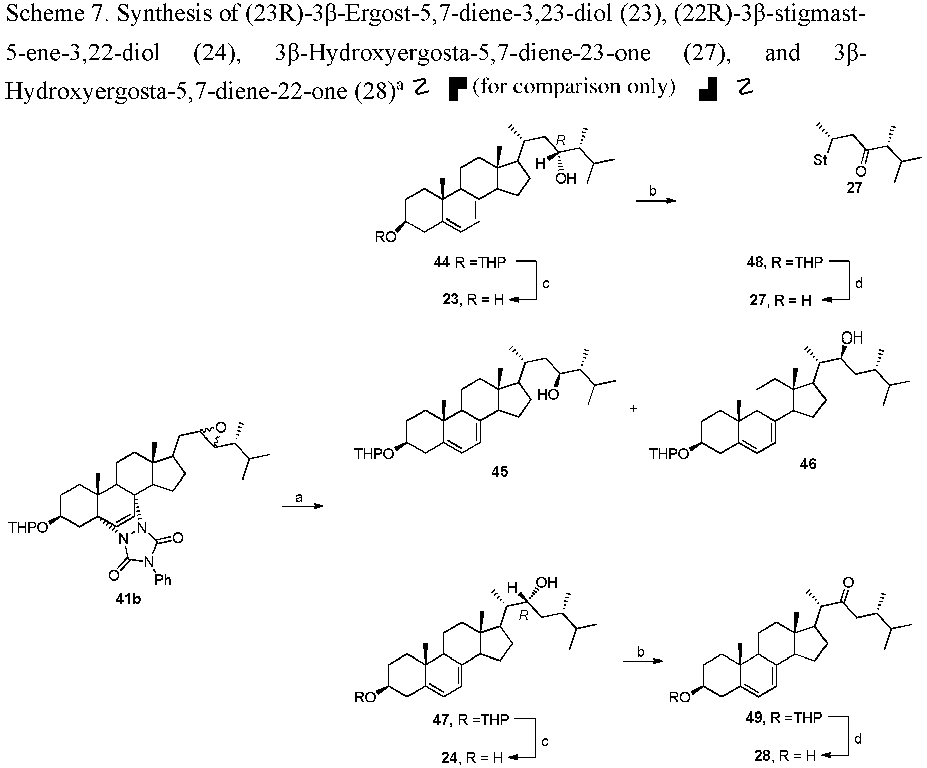 ^{a}Reagents and conditions: (a) LiAlH₄, THF-Et₂O, reflux, 36 h; (b) (COCl)₂, DMSO, CH₂Cl₂, -78°C, 2h, then Et₃N, r.t.; (c) PPTS, EtOH, reflux, 1h; (d) PPTS, acetone, reflux, 5h.

Subsequent Swern oxidation of the single alcohol 44 afforded the 3β-tetrahydropyranyl-23-keto derivative 48, which was deprotected under analogous mild acidic conditions to finally afford 3β-hydroxyergosta-5,7-diene-23-one (27). An analogous sequence, starting from the more polar, pure 22R-hydroxy derivative 47 gave access to the desired (22R)-3β-ergost-5,7-diene-3,22-diol (24) and 3β-hydroxyergosta-5,7-diene-22-one (28).

The ergostanediol series was completed by reducing the 3β-tetrahydropyranyl-23-keto derivative 48 with sodium borohydride, achieving almost quantitatively the mixture of the two 23-hydroxy epimers, which, after deprotection at C-3 position, gave the already obtained (23R)-3β-ergost-5,7-diene-3,23-diol (23), and the missing 23S-epimer 21 (Scheme 8). Analogously, starting from the 22-keto derivative 49, (22S)-3β-ergost-5,7-diene-3,22-diol (22) was achieved along with the already obtained 24

(Scheme 9), however, none of these compounds nor their indication for any therapeutic use forms part of the present invention. 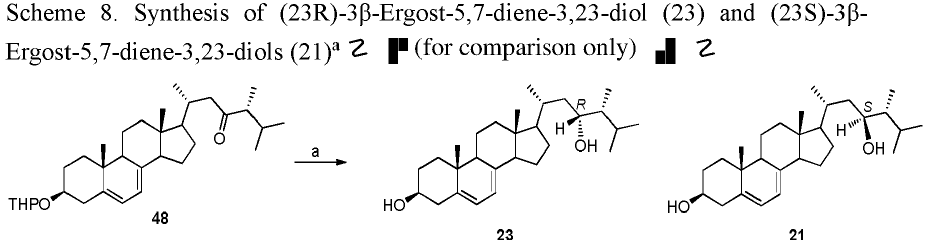 ^{a}Reagents and conditions: (a) *i.* NaBH₄, THF, 2-propanol, H₂O, r.t.; *ii.* PPTS, EtOH, reflux, 1h; *iii.* mpc. 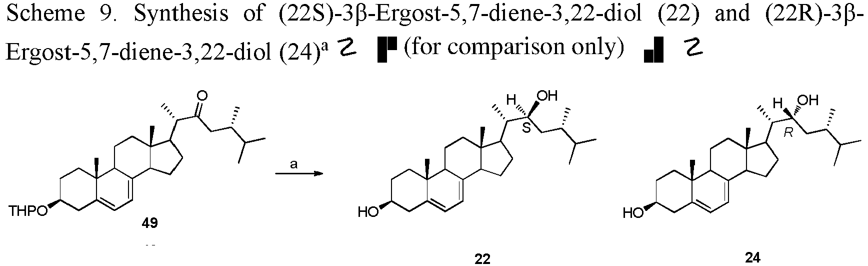 ^{a}Reagents and conditions: (a) *i.* NaBH₄, THF, 2-propanol, H₂O, r.t.; *ii.* PPTS, EtOH, reflux, 1h; *iii.* mpc. 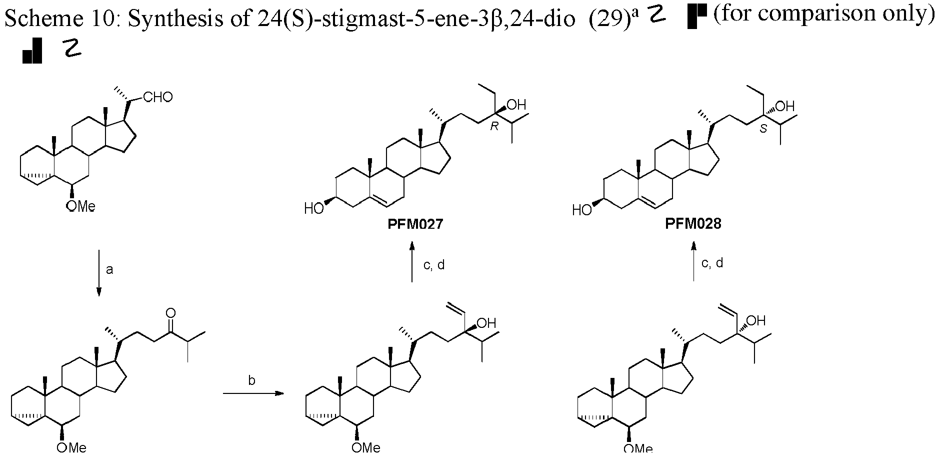 ^{a} Reagent and conditions: (a) *i.* Ph3P=CHC(O)CH(CH3)2, toluene, 92 h, reflux; *ii.* H2, 40 psi, 10% Pd/C, NaHCO₃, EtOAc/THF, 30 h, r.t.; 60% yield. (b) 1M CH2=CHMgBr in THF, Et2O, 2 h, reflux; *ii.* mpc, 79% yield. (c) pTosOH·H2O, 75% aqueous dioxane, 75 °C, 1 h; (d) H2, 40 psi, 10% Pd/C, NaHCO3, EtOAc/THF, 12 h, r.t.

In the above process of scheme 10, 40psi = 275790Pa.

### Absolute Configuration Assignment

The workflows for the stereochemical elucidation of the newly created asymmetric centers are depicted in Figures 1 and 2.

In the case of the members of the stigmastane series it was used the X-ray single crystal diffraction analysis reported for (22R)-3β-stigmast-5-ene-3,22-diol, the only known derivative among the stigmastanediols here reported.39,40 By comparison of its reported spectroscopic data with those of our compounds, it was established that the more polar diol 20 corresponded to (22R)-3β-stigmast-5-ene-3,22-diol. Since 20 had been obtained from the reductive opening of the more polar oxirane isomer 14, as a consequence, the latter had to be endowed with the 22S,23S-absolute configuration. Thus, the other diol deriving from its reductive opening, namely 19, was assigned instead with the 23R-configuration (Figure 1). By exclusion, the diols 17 and 18 were characterized by the S-configuration at the newly formed side-chain chiral center, and the less polar epoxide 13 by 22R,23R-configuration. The respective position of the hydroxyl group in the two diols 17 and 18 was definitively established by their comparison with the compounds resulting from the reduction of 3β-hydroxystigmast-5-ene-22-one (26).

Although the synthesis of some of our ergostane derivatives had been already reported, their structural assignment had been only presumed.36,41,42 To unambiguously proceed with the structural elucidation, the diol 23, derived by the hydrolysis of 44, the less polar, major fraction obtained by the reductive opening of the epoxide mixture 41b (Scheme 7), was submitted to single crystal X-ray analysis (Figure 1) and thus characterized as the (23R)-isomer. Consequently, the diol 24, since obtained by the hydrolysis of the other more abundant isomer 47 resulting from the same opening reaction (Scheme 7), was assigned as (22R)-3β-ergost-5,7-diene-3,22-diol (Figure 2). Since these two major isomers surely derived from the opening of a unique epoxide, the absolute configuration 22S,23S was assigned to the more abundant epoxide 16, and consequently, the 22R,23R-configuration to the less abundant 15. The diol obtained by the reduction of the 23-keto derivative 27, different from 23, had to be the (23S)-isomer 21, as well as the other diol deriving from the 22-keto derivative 28 and distinct from 24, was the 22S-derivative 22.

Other preferred compounds for use according to the present invention, are those listed in Table (I).

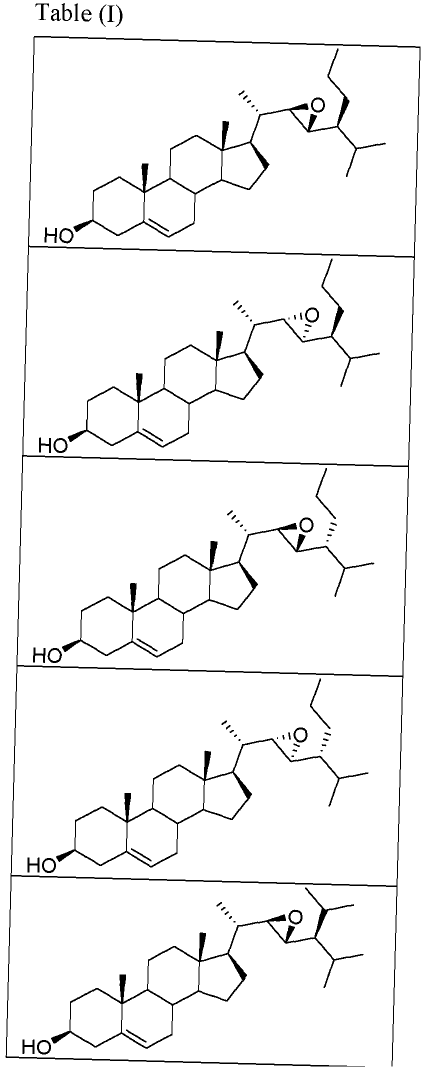

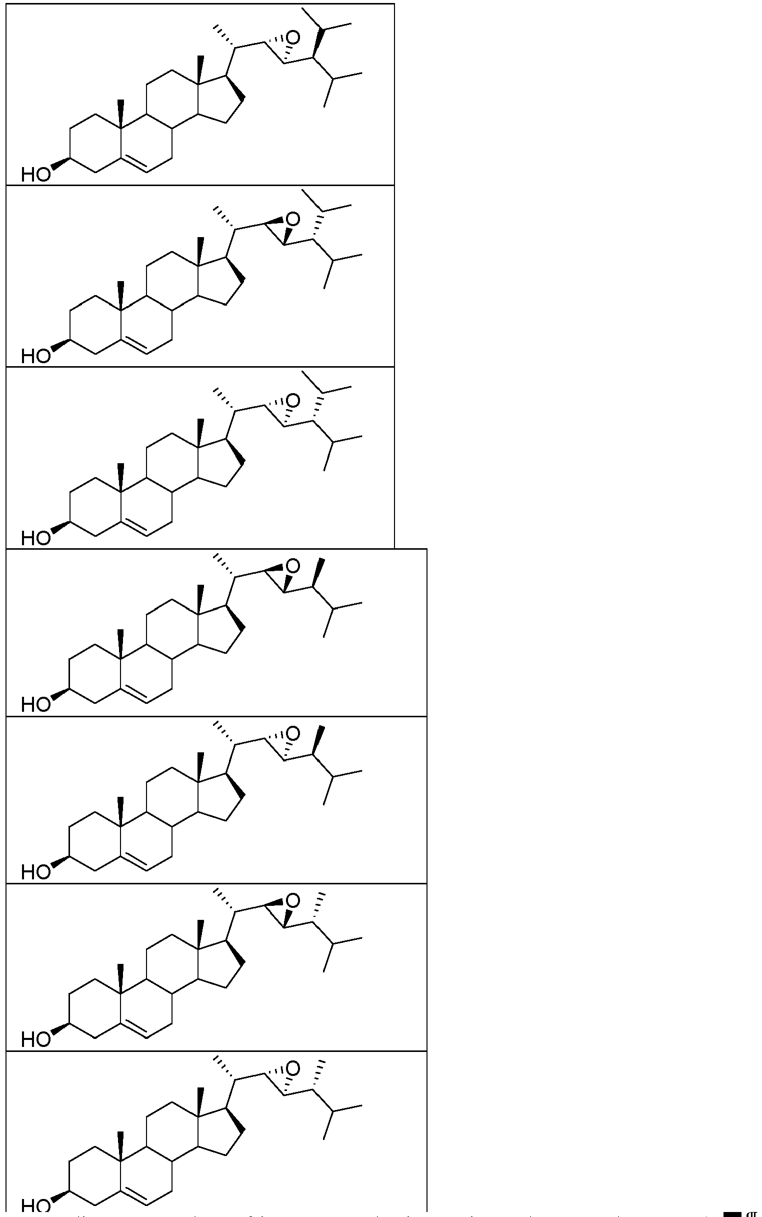

The compounds of formula (I) and especially the preferred compounds, are LXR modulators, more particularly LXR agonists
According to another of its aspect, the invention relates to the compounds of formula (I) and especially of the preferred compounds, for use in therapy.

More particularly, the above compounds are useful in the prevention and/or treatment of diseases linked to LXR, such as, but not limited to, cancer, inflammatory and/or immunological diseases. Particularly preferred are the use in the prevention and/or treatment of melanoma, prostate cancer and skin carcinoma. Also preferred are chronic inflammatory diseases, metabolic diseases and autoimmune diseases.

For their use in therapy, the compounds of the invention are formulated in pharmaceutical compositions along with at least one pharmaceutically acceptable carrier according to the methods known in the art; said compositions constitute another aspect of the present invention.

The skilled in the art is aware of the type of carrier to choose, based upon the intended route of administration. The compositions of the invention are suitable for intravenous, intraperitoneal, subcutaneous, intramuscular, topical, transdermal or oral administration. Pharmaceutically acceptable carriers include sterile aqueous solutions or dispersions and sterile powders for the extemporaneous preparation of sterile injectable solutions or dispersion.

The compositions for use according to the invention may also comprise additional active ingredients.

According to another of its aspects, the invention relates to the compounds of formula (I) for use in a method for the prevention and/or the treatment of a disease linked to LXR, which comprises administering to a mammal in need thereof, an effective amount of at least one compound of formula (I). A preferred embodiment, according to the invention, relates to the compounds of formula (I) for use in a method for the prevention and/or treatment of cancer, inflammatory and/or immunological diseases. Particularly preferred are the methods in the prevention and/or treatment of melanoma, prostate cancer and skin carcinoma. Also preferred are chronic inflammatory diseases, metabolic diseases and autoimmune diseases.

According to another of its aspects, the invention relates to synthesis intermediate compounds having the following formulas 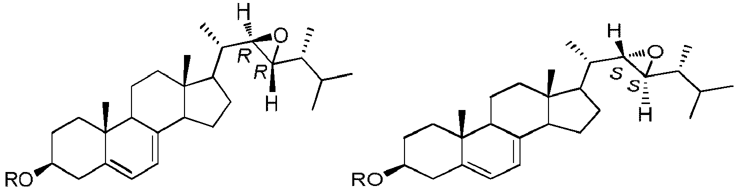 wherein R is an acetyl group.

The invention is illustrated in the Experimental Section which follows, in a nonlimiting way.

### Experimental Section

The following experimental section relates to the subject matter of the present invention only insofar as it relates to the therapeutic activity of 22,23-epoxy compounds 13-16 and the compounds 42 and 43 *per se.*

### LXRs Activity

All the synthesized compounds were first tested for their ability to activate LXRs by using luciferase assays with GAL-4 chimeric receptors. These were performed by co-transfecting plasmids encoding hLXRa- and β-binding domains fused to GAL-4, with the respective responsive element conjugated with the luciferase reporter gene into the human embryonic kidney 293 cells. Results of the assays are listed in Table 2: most of the compounds exhibited low micromolar LXRs activity retaining or, in some cases, improving the magnitude of activity of the endogenous ligand 22R-HC (3).

**Table 2. LXR Agonist Profile of Compounds 13-28**

| Compd | LXRα EC₅₀ (µM)^{a} ± SD (95% C.I.)^{b} | Efficacy (%)^{b} ± SD | LXRβ EC₅₀ (µM)^{a} ± SD (95% C.I.)^{b} | Efficacy (%)b ± SD |
|---|---|---|---|---|
| 22*R-*HC (3) | 6.71 ± 0.71 (5.4 - 8.2) | 100 | 4.75 ± 0.12 (3.4 - 6.4) | 100 |
| 13 | 2.09 ± 0.57 (1.1 - 3.4) | 488.8 ± 89.1 | 3.79 ± 0.82 (2.5 - 5.6) | 142.1 ±16.8 |
| 14 | 16.43 ± 0.62 (13.1 - 24) | 56.8 ± 4.6 | 12.72 ± 2.7 (11.6 - 14.9) | 36.3 ± 3.0 |
| 15 | 4.11 ± 0.31 (3.5-4.7) | 93.2 ± 5.8 | 7.2 ± 0.94 (0.1 - 15.7) | 30.4 ± 10.6 |
| 16 | 1.5 ± 0.12 (1 - 2.1) | 51.7 ± 3.8 | 1.96 ± 0.05 (1.0 - 2.3) | 48.5 ± 13 |
| 19* | 3.2 ± 0.54 (2.0 - 4.7) | 489.3 ± 70.1 | 2.7 ± 1.16 (1.8 - 3.9) | 115.1 ± 2.8 |
| 20 * | 6.93 ± 1.9 (2.1 - 11.8) | 208.3 ± 69.3 | 2.31 ± 0.36 (0.3 - 14.6) | 90.8 ± 13.2 |
| 21* | 8.07 ± 1.60 (7.7 - 8.8) | 159.3 ± 41.3 | NA^{c} | - |
| 22* | 6.61 ± 1.69 (4 - 8.7) | 74.7 11.1 | 1.96 ± 0.1 (0.6 - 6.5) | 41.9 ± 19.2 |
| 23* | 15.75 ± 0.65 (14.5 - 17) | 63.9 ± 26.9 | NA^{c} | - |
| 25 * | 14.51 ± 1.86 (7.4 - 23.2) | 12.4 ± 4.2 | 6.02 ± 1.2 (4.7 - 7.5) | 46.4 ± 8.5 |
| 27 * | 5.58 ± 0.30 (4.6 - 6.4) | 150.6 ± 4.8 | NA ^{c} | - |
| 28 * | 8.51 ± 0.42 (7.5 - 9.7) | 70.2 ± 3.9 | NA^{c} | - |
| 29* | 3.72 ± 0.5 (1.85 - 6.49) | 11.04 ± 4.08 | 3.21 ± 0.6 (0.89 - 8.64) | 48.37 ± 5.0 |
| 12* | 2.92 ± 0.05 (1.03 - 7.07) | 43.44 ± 17.65 | 1.69 ± 0.22 (0.66 - 5.13) | 61.04 ± 17.36 |

| | | | | |
|---|---|---|---|---|
| ^{∗} not according to the invention ^{a}Fifty% maximal activation (EC₅₀) ± standard deviations (SD) was determined by dose-response curve of titrating concentrations of compounds **13-28** (32, 16, 8, 4, 2 and 1 µM) tested by luciferase assays. The results were mean of three-five independent experiments; ^{b}Efficacy: % of compound effect ± SD versus 8 µM of 22R-HC; ^{c}NA: not active. | | | | |

Concerning the isoform selectivity profile, besides non-selective and poorly preferential LXRα agonists (13, 16 and 19) (Figures 6-9), other compounds, such as 21, 27 and 28, deserve to be highlighted as selective LXRα agonists. Among them, the derivative 27 showed to be the most promising α-selective agonist thanks to its lower EC50 value and higher efficacy respect to the reference compound 3.

Furthermore, 20, 22 and 25 and 29 showed a good selectivity for the LXRβ isoform in terms of EC50.

The reported compounds **25** and **29** showed to be endowed with improved isoform selectivity in comparison to the known derivative **12.**

The beta/alfa efficacy ratios for compounds **25** and **29,** indeed, were 3.7 and 4.4, respectively, respect to the value of 1.4 for **12.**

In particular, 25 can be considered a LXRβ-selective agonist, being virtually inactive (EC50 = 14.51 µM) at LXRα (Figures 7 and 9). Of note, 25 while showing approximately 50% of efficacy in terms of LXRβ activation, was endowed with the lowest efficacy at LXRα, as compared to the 22R-HC (3) (Table 2); Thus, confirming its selectivity for the LXRβ isoform. From a structural point of view, all the selective LXRα agonists are ergostane derivatives, whereas the preferential LXRβ ligands belong to the two classes; however the most interesting compound in this sense, namely 25, is a stigmastane derivative.

Moreover, the skeleton system more than the nature and, where applicable, the stereochemistry of the side-chain modification, appeared to strictly influence both potency and isoform selectivity. Indeed, with R,R-epoxy derivatives 13 and 15, as the only exceptions, any equally side-chain modified ergostane and stigmastane derivatives did not show similar biological profile.

The selectivity of our compounds within the nuclear receptor superfamily was also evaluated by luciferase assays using GAL4-RXR, -PPARγ, -PXR and -FXR plasmids. No compound was able to activate RXR or PPARγ, whereas a slight activation of PXR by 21, 22 and 15, and a strong FXR activation by 20 was observed (Figure 10).

### Gene expression profile

LXR agonists induce the expression of target genes, which are involved in cholesterol homeostasis, particularly in the reverse cholesterol transport pathway.43 Indeed, LXR agonists induce the expression of ABCA1 both in macrophages and in many tissues of the periphery such as the intestine.44 Moreover, ABCA1 regulates cholesterol efflux to APOAI acceptors.45 In the liver, LXR activation promotes the biosynthesis of fatty acids, a process also termed as de novo lipogenesis by inducing the expression of the master regulator of hepatic lipogenesis sterol-regulatory element-binding protein 1C (SREBP-1c), as well as several downstream genes in the SREBP-1c pathway, including steroyl CoA desaturase 1 (SCD1) and fatty acid synthase (FASN).43 Therefore, it was investigated, by quantitative PCR (qPCR), the expression of ABCA1, SREBP1c, FASN, and SCD1, by using RNA from monocytic U937 cells (Figure 3) and from hepatic HepG2 cells (Figure 4) stimulated with our compounds, the non-steroidal agonist T0901317 or the endogenous ligand 22R-HC (3) as positive controls. As shown in Figure 3A, all the compounds, except 18 and 15, were able to induce ABCA1 expression, although to a different extent. With most derivatives, a mild up-regulation of the gene expression (2 fold) was observed, whereas, interestingly, with 13, 19, 20 and 25 a strong induction of ABCA1 expression was detected, comparable to that caused by T0901317. Noteworthy, for all our compounds the level of up-regulation of SREBP-1c was much lower than that observed for T0901317 and comparable to the level obtained with the natural ligand 22R-HC (3) (Figure 3B). The effects observed on FASN and SCD1 genes were even more interesting: no compound up-regulated the mRNA levels of FASN (Figure 3C); a slight activation (below 2-fold) of SCD1 was detected only for 16 and 25, with the latter being statistically significant (Figure 3D). Also the natural ligand 22R-HC (3) did not induce up-regulation of FASN and SCD1 transcripts (Figures 3C and 3D). These data were confirmed at later time points (i.e. 16 hours). Then, the induction of genes involved in the lipogenesis using the hepatic cell line HepG2. 43,22 was evaluated. By qPCR analysis it was observed only a significant up-regulation of SREBP-1c induced by 13 and 16 compounds, while all the other compounds turned out to be negative (Figure 4A). No compound up-regulated the mRNA levels of FASN (Figure 4B) and SCD1 (Figure 4C). According to all these evidences, the derivatives 13, 19, 20 and 25, being strong inducers of ABCA1, poor activators of SREBP1c and SCD1 in the U937 cell line, showed to be very promising derivatives. Over the time, indeed, substantial efforts have been dedicated to the identification of LXR ligands able to turning on ABC transporter genes, without affecting lipogenic genes levels. This task is still one of the major challenge to the discovery of a clinically useful LXR modulator for atherosclerosis. According to the isoform selectivity profile, 13 and 19 were non-selective ligands, 20 and 25 were a preferential and a selective LXRβ agonist, respectively, thus evidencing that in our model the ability to not up-regulate the genes involved in lipogenesis was not a phenomenon exclusive of LXRβ-selective modulators.

Being LXRs not only transcriptional regulators of the cholesterol and lipid homeostasis, but also able to exert potent anti-inflammatory effects through the interference of TLRs 2, 4 and 9 signaling,46 it was decided to verify whether our compounds were also capable of modulating genes involved in the inflammatory pathways, such as the MCP-1/CCL2 and TNFα genes, which have been shown to be inhibited when LXRs are engaged in the presence of LPS.47 To this purpose, differentiated U937 cells was treated for 6 hours with our compounds in combination with LPS (100 ng/ml) and then the treated cells for the expression of CCL and TNFα by qPCR was evaluated. Most of the compounds were able to inhibit CCL2 expression with 21, 22, 19 and 16 showing the same grade of potency of the positive control T0901317 (Figure 5A). Most of the compounds were also able to inhibit TNFα with 21, 13, 19 and 25 being the most active (Figure 5B). Similar results were obtained by using the endogenous ligand 22R-HC (3).

### Chemistry.

Melting points were determined by the capillary method on a Büchi 535 electrothermal apparatus and are uncorrected. 1H- and 13C NMR spectra were taken on a Bruker AC 400 spectrometer as solutions in CDCl3 unless otherwise indicated. The spin multiplicities are indicated by the symbols s (singlet), d (doublet), t (triplet), q (quartet), m (multiplet), and bs (broad). Flash chromatography was performed on Merck silica gel (0.040-0.063 mm). Medium pressure chromatography (mpc) was performed on Merck LiChroprep Si 60 Lobar columns. Microanalyses were carried out on a Carlo Erba 1106 elemental analyzer and the results were within ± 0.4% of the theoretical values. All solvents were distilled and dried according to standard procedures. Purity was determined by microanalysis to be >95% for all final compounds.

### Example 1

(22R,23R)-22,23-Epoxystigmast-5-ene-3β-ol (13). The epoxide 30 (0.067 g, 0.15 mmol) was refluxed in glacial acetic acid (5 mL) for 5 h. The residue obtained by the removal of the solvent in vacuo was directly dissolved in methanol/water (2:1, 12 mL) and the resulting solution treated with K2CO3 (0.26 g, 1.86 mmol) and refluxed for 3 h. After cooling the reaction mixture was extracted with CH2Cl2 (3x10 mL) and the combined organic layers dried over Na2SO4. The solvent was removed in vacuo and the residue submitted to mpc. Elution by light petroleum-ethyl acetate (80:20) afforded pure sample of 13: 36% yield; mp 173.2-175.4 °C; 1H NMR (400 MHz) δ 0.69 (s, 3H), 2.28-2.29 (m, 2H), 2.49-2.50 (m, 1H), 2.75 (dd, 1H, J = 9.32 and 2.21 Hz), 3.52 (m, 1H), 5.35-5.36 (m, 1H); 13C NMR (100 MHz) δ 11.82, 12.45, 16.17, 19.37, 19.54, 20.17, 20.85, 21.01, 24.53, 27.93, 29.13, 31.61, 31.88 (2C), 36.48, 37.22, 38.66, 39.55, 42.24, 42.62, 48.28, 50.07, 53.42, 56.35, 62.14 (2C), 71.74, 121.54, 140.79; Anal. Calcd for C29H48O2: C, 81.25%; H, 11.29%. Anal. Found: C, 81.17%; H, 11.24%.

### Example 2

(22S,23S)-22,23-Epoxystigmast-5-ene-3β-ol (14). The epoxide 31 was treated as reported for compound 30 to furnish 14 in 29% yield; mp 127.8-130.2 °C; 1H NMR (400 MHz) δ 0.68 (s, 3H), 2.24-2.30 (m, 2H), 2.49-2.54 (m, 2H), 3.53 (m, 1H), 5.35-5.37 (m, 1H); 13C NMR (100 MHz) 11.97, 12.36, 16.28, 19.36 (2C), 20.92, 21.06, 24.51, 27.07, 29.31, 29.68, 31.62, 31.87 (2C), 36.48, 37.25, 38.87, 39.67, 42.27, 42.67, 48.77, 50.17, 56.02, 56.32, 58.55, 63.13, 71.77, 121.67, 140.67; Anal. Calcd for C29H48O2: C, 81.25%; H, 11.29%. Anal. Found: C, 81.33%; H, 11.27%.

### Example 3

(22R,23R)-22,23-Epoxyergosta-5,7-diene-3β-ol (15). 2M KOH solution (0.2 mL) was added to a solution of 42 (0.037 g, 0.08 mmol) in EtOH (3.8 mL) and the resulting mixture was refluxed for 15 min. After cooling the reaction mixture was extracted with EtOAc (4x5 mL) and the combined organic layers were washed with brine (8 mL), dried over Na2SO4, filtered and the solvent removed in vacuo to give a residue which was submitted to flash chromatography. Elution with light petroleum-ethyl acetate (80:20) afforded 15 in 64% yield; mp: 163.8-165.2 °C; 1H NMR (400 MHz) δ 0.61 (s, 3H), 3.61-3.65 (m, 1H), 5.39-5.41 (m, 1H), 5.57-5.58 (m, 1H); 13C NMR (100 MHz) 11.9, 13.7, 16.2, 16.3, 19.5, 20.4, 21.0, 23.2, 26.8, 31.1, 31.9, 37.0, 38.3, 39.0 (2C), 40.7, 42.3, 43.2, 46.2, 54.0, 55.6, 60.4, 64.3, 70.3, 116.5, 119.5, 139.8, 140.8; Anal. Calcd for C28H44O2: C, 81.50%; H, 10.76%. Anal. Found: C, 81.17%; H, 10.74%.

### Example 4

(22S,23S)-22,23-Epoxyergosta-5,7-diene-3β-ol (16). The derivative 43 was treated as reported for 42 to furnish 16 in 89% yield: mp: 138.3-139.6 °C; 1H NMR (400 MHz) δ 0.60 (s, 3H), 3.60-3.66 (m, 1H), 5.39-5.41 (m, 1H), 5.56-5.58 (m, 1H); 13C NMR (100 MHz) 11.8, 12.5, 16.2, 17.1, 18.5, 20.2, 21.0, 23.3, 27.8, 31.0, 31.9, 37.0, 38.3, 39.0, 39.8, 40.7, 42.5, 43.2, 46.1, 53.3, 54.0, 63.1, 63.8, 70.3, 116.5, 119.4, 140.0, 140.7; Anal. Calcd for C28H44O2: C, 81.50%; H, 10.76%. Anal. Found: C, 81.32%; H, 10.77%.

### Example 5 (not according to the invention)

(23S)-3β-Stigmast-5-ene-3,23-diol (17) and (22S)-3β-Stigmast-5-ene-3,22-diol (18).

LiAlH4 (0.25 g, 6.71 mmol) was portion wise added to the solution of the epoxide 30 (0.27 g, 0.61 mmol) in anhydrous THF (15 mL). The resulting mixture was refluxed for 36 h under an argon atmosphere. After cooling, first EtOAc and then water were carefully added. The organic phase was separated and the water phase extracted with EtOAc (3x15 mL). The combined organic phases were washed with brine (20 mL) and then dried over Na2SO4. After filtration, the solvent was evaporated in vacuo to give a residue, which was dissolved in glacial acetic acid (5 mL) and the resulting solution refluxed for 6 h. After cooling, the mixture of 32 + 33, obtained by the removal of the solvent in vacuo, was directly dissolved in methanol (16 mL) and treated with 2M KOH solution (8 mL). After refluxing for 3 h, the reaction mixture was extracted with EtOAc (3 × 15 ml). The combined organic layers were dried over Na2SO4, filtered and the solvent removed in vacuo, to give a residue which was submitted to mpc. Elution by light petroleum-ethyl acetate (70:30) afforded pure samples of the desired compounds in 69% total yield; 17: mp 178.2-181.4 °C; 1H NMR (400 MHz) δ 0.69 (s, 3H), 2.23-2.31 (m, 2H), 3.53 (m, 1H), 3.91 (m, 1H), 5.36 (m, 1H); 13C NMR (100 MHz) 11.79, 13.82, 18.28, 19.38 (2C), 19.85, 21.03, 21.12, 24.24, 28.45, 28.54, 31.58, 31.82 (2C), 34.16, 36.44, 37.19, 39.73, 42.22, 42.35, 42.46, 49.13, 50.01, 56.66, 56.88, 70.55, 71.73, 121.62, 140.72; Anal. Calcd for C29H50O2: C, 80.87%; H, 11.70%. Anal. Found: C, 80.63%; H, 11.72%; 18: mp 168.9-172.4 °C; 1H NMR (400 MHz) δ 0.70 (s, 3H), 2.24-2.31 (m, 2H), 3.53 (m, 1H), 3.75 (t, 1H, J = 6.77 Hz), 5.35 (d, 1H, J = 5.21 Hz); 13C NMR (100 MHz) 11.39, 11.73, 11.85, 18.89, 19.10, 19.38, 21.05, 23.27, 24.18, 27.8, 28.84, 31.58, 31.80, 31.88, 35.77, 36.43, 37.20, 39.72, 39.92, 42.04, 42.20 (2C), 50.02, 52.58, 56.61, 71.71, 71.86, 121.60, 140.73; Anal. Calcd for (C29H50O2): C, 80.87%; H, 11.70%. Anal. Found: C, 80.70%; H, 11.65%.

### Example 6 (not according to the invention)

(23R)-3β-Stigmast-5-ene-3,23-diol (19). A solution of 36 (0.03 g, 0.06 mmol) in MeOH (3 mL) was treated with 2M KOH solution (1 mL) and the resulting mixture was refluxed for 30 min. After cooling the reaction mixture was extracted with EtOAc (3x10 mL) and the combined organic layers were dried over Na2SO4, filtered and the solvent removed in vacuo. The residue, thus obtained, was purified by flash chromatography: elution with light petroleum-ethyl acetate (80:20) afforded 19 in 55% yield: mp 158.1-158.6 °C; 1H NMR (400 MHz) δ 0.72 (s, 3H), 2.27-2.28 (m, 2H), 3.51 (m, 1H), 3.69-3.74 (m, 1H), 5.35 (d, 1H, J = 5.25 Hz); 13C NMR (100 MHz) 11.94, 14.48, 18.63, 18.96, 19.17, 19.36, 21.09, 21.44, 24.25, 27.80, 28.50, 31.67, 31.90 (2C), 32.78, 36.51, 37.28, 39.87, 41.11, 42.32, 42.51, 50.17, 52.49, 56.92 (2C), 70.25, 71.76, 121.60, 140.81; Anal. Calcd for C29H50O2: C, 80.87%; H, 11.70%. Anal. Found: C, 80.67%; H, 11.66%.

### Example 7 (not according to the invention)

(22R)-3β-stigmast-5-ene-3,22-diol (20). The derivative 37 was treated as reported for compound 36 to furnish 20 in 72% yield: mp: 149.2-149.9 °C; 1H NMR (400 MHz) δ 0.72 (s, 3H), 3.51 (m, 1H), 3.69-3.74 (m, 1H), 5.35 (d, 1H, J = 5.25 Hz); 13C NMR (100 MHz) 11.75 (2C), 12.32, 17.74, 19.38, 20.45, 21.11, 23.60, 24.45, 27.50, 28.92, 29.65, 30.11, 31.70, 31.92, 36.54, 37.30, 39.81, 41.53, 42.33, 42.59, 42.70, 50.22, 53.07, 56.39, 71.39, 71.78, 121.59, 140.85; Anal. Calcd for C29H50O2: C, 80.87%; H, 11.70%. Anal. Found: C, 80.91%; H, 11.69%.

### Example 8 (not according to the invention)

(23R)-3β-Ergost-5,7-diene-3,23-diol (23) and (23S)-3β-Ergost-5,7-diene-3,23-diol (21). NaBH4 (0.13 g, 3.44 mmol) was added to a solution of the ketone 48 (0.10 g, 0.2 mmol) in THF-2-propanol (2:1, 6 mL) and the resulting mixture was stirred at room temeprature overnight. The reaction mixture was then diluted with H2O (5 mL) and extracted with Et2O (3x5 mL). The combined organic layers were washed with brine (10 mL), dried over Na2SO4, filtered and the solvent removed in vacuo. The residue, thus obtained, was dissolved in EtOH (10 mL) and treated with PPTS (0.012 g, 0.047 mmol). After refluxing for 1 h, the reaction mixture was allowed to cool to room temperature, and the solvent was removed in vacuo to give a residue, which was submitted to mpc. Elution with light petroleum-ethyl acetate (90:10) afforded pure samples of the desired compounds in 78% total yield; 21: mp 129.8-130.7 °C; 1H NMR (400 MHz) δ 0.65 (s, 3H), 3.62-3.69 (m, 2H), 5.40-5.42 (m, 1H), 5.59 (dd, 1H, J = 7.89, 2.35 Hz). 13C NMR (100 MHz) 10.54, 11.71, 16.27, 17.92, 20.67, 21.04, 21.75, 23.05, 27.74, 28.40, 31.92, 35.75, 36.97, 38.32, 39.09, 40.70 (2xC), 43.00, 45.32, 46.16, 54.37, 56.76, 70.44, 73.30, 116.33, 119.54, 139.80, 141.24; Anal. Calcd for C28H46O2: C, 81.10%; H, 11.18%. Anal. Found: C, 80.97%; H, 11.19%.

### Example 9 (not according to the invention)

(22R)-3β-Ergost-5,7-diene-3,22-diol (24) and (22S)-3β-Ergost-5,7-diene-3,22-diol (22). The derivative 49 was treated as reported for 48 to furnish pure samples of the desired compounds 24 and 22 in 83% total yield. 22: mp 117.3-121.0 °C; 1H NMR (400 MHz) δ 0.65 (s, 3H), 0.79 (d, 3H, J = 6.84 Hz), 3.63-3.66 (m, 1H), 3.78-3.81 (m, 1H), 5.40-5.42 (m, 1H), 5.59-5.61 (m, 1H); 13C NMR (100 MHz) 11.79, 12.49, 15.55, 16.02, 16.22, 21.04, 21.12, 23.15, 23.80, 27.39, 29.53, 31.92, 34.60, 35.27, 36.98, 38.30, 39.09, 40.72, 43.00, 46.14, 52.73, 54.01, 70.40, 71.67, 116.43, 119.54, 139.90, 140.96; Anal. Calcd for C28H46O2: C, 81.10%; H, 11.18%. Anal. Found: C, 81.09%; H, 11.17%.

### Example 10 (not according to the invention)

(23R)-3β-Ergost-5,7-diene-3,23-diol (23). PPTS (0.010 g, 0.039 mmol) was added to a solution of 44 (0.050 g, 0.1 mmol) in EtOH (5 mL) and the resulting mixture was refluxed for 5 h. After cooling the solvent was removed in vacuo and the residue was purified by flash chromatography. Elution with light petroleum-ethyl acetate (80:20) furnished 23 in 70% yield: mp 167.8-169.4 °C; 1H NMR (400 MHz) δ 0.68 (s, 3H), 3.68 (m, 1H), 3.82 (m, 1H), 5.44 (s, 1H), 5.61 (s, 1H). 13C NMR (100 MHz) 9.83, 11.88, 16.25, 18.45, 18.79, 21.05, 21.50, 22.98, 28.33, 29.55, 31.92, 33.11, 36.97, 38.33, 39.18, 40.73, 42.11, 43.01, 45.37, 46.17, 54.52, 56.47, 70.38, 70.64, 116.34, 119.53, 139.80, 141.19; Anal. Calcd for C28H46O2: C, 81.10%; H, 11.18%. Anal. Found: C, 81.26%; H, 11.16%.

### Example 11 (not according to the invention)

(22R)-3β-Ergost-5,7-diene-3,22-diol (24). The derivative 47 was treated as reported for 44 to furnish 24 in 73% yield: mp 197.7-201.2 °C; 1H NMR (400 MHz) δ 0.65 (s, 3H), 0.79 (d, 3H, J = 6.84 Hz), 3.63-3.66 (m, 1H), 3.78-3.81 (m, 1H), 5.40-5.42 (m, 1H), 5.59-5.61 (m, 1H); 13C NMR (100 MHz) 11.79, 12.49, 15.55, 16.02, 16.22, 21.04, 21.12, 23.15, 23.80, 27.39, 29.53, 31.92, 34.60, 35.27, 36.98, 38.30, 39.09, 40.72, 43.00, 46.14, 52.73, 54.01, 70.40, 71.67, 116.43, 119.54, 139.90, 140.96; Anal. Calcd for C28H46O2: C, 81.10%; H, 11.18%. Anal. Found: C, 80.86%; H, 11.20%.

### Example 12 (not according to the invention)

3β-Hydroxystigmast-5-ene-23-one (25). A solution of DMSO (0.03 g, 0.38 mmol) in anhydrous CH2Cl2 (0.5 mL) was added to a solution of oxalyl chloride (0.025 g, 0.2 mmol) in anhydrous CH2Cl2 (1 mL), kept at -60 °C under an argon atmosphere. After the resulting mixture was stirred for 15 min at -60 °C, the solution of alcohol 36 (0.048 g, 0.1 mmol) in anhydrous CH2Cl2 (1 mL) was added. The mixture was stirred for 2 h at -55/60 °C before the addition of Et3N (0.08 g, 0.76 mmol). After the reaction mixture was allowed to reach room temperature, stirring was continued for 15 min, and then water (10 mL) was added. The reaction mixture was extracted with CH2Cl2 (3x5 mL), the combined organic layers were washed with brine (10 mL), dried over Na2SO4, filtered and the solvent removed in vacuo, to give the crude ketone 38, which was dissolved in acetone (2 mL) and treated with 2M KOH solution (0.5 mL). The resulting solution was refluxed for 40 min, cooled and extracted with EtOAc (3x5 mL). The combined organic layers were dried over Na2SO4, filtered and the solvent removed in vacuo to give a residue then submitted to flash chromatography. Elution with light petroleum-ethyl acetate (80:20) furnished 25 in 51% yield: mp: 174.2-174.8 °C; 1H NMR (400 MHz) δ 0.62 (s, 3H), 2.40-2.42 (m, 2H), 3.40-3.47 (m, 1H), 5.26 (d, 1H, J = 4.9 Hz); 13C NMR (100 MHz) 11.77, 12.02, 16.57, 18.46, 19.37, 19.61, 21.04, 23.90, 24.52, 27.64, 28.93, 31.64, 31.83, 31.90, 36.50, 37.27, 39.66, 39.82, 42.29, 42.47, 43.27, 49.66, 50.12, 51.95, 56.11, 71.72, 121.51, 140.78, 214.50; Anal. Calcd for C29H48O2: C, 81.25%; H, 11.29%. Anal. Found: C, 81.32%; H, 11.31%.

### Example 13 (not according to the invention)

3β-Hydroxystigmast-5-ene-22-one (26). The derivative 37 was treated as reported for the compound 36 to furnish 26 in 59% yield: mp: 151.7-152.8 °C; 1H NMR (400 MHz) δ 0.72 (s, 3H), 3.48-3.53 (m, 1H), 5.32-5.33 (m, 1H); 13C NMR (100 MHz) 11.85, 19.35, 19.70, 20.06, 21.18, 21.57, 24.22, 28.37, 29.17, 31.62, 31.71, 31.85, 36.47, 37.24, 39.66, 42.26, 42.42, 50.06, 51.04, 55.70, 56.82, 60.82, 71.73, 121.52, 140.81, 214.58; Anal. Calcd for C29H48O2: C, 81.25%; H, 11.29%. Anal. Found: C, 81.49%; H, 11.28%.

### Example 14 (not according to the invention)

3β-Hydroxyergost-5,7-diene-23-one (27). PPTS (0.010 g, 0.039 mmol) was added to a solution of 48 (0.050 g, 0.1 mmol) in acetone (5 mL) and the resulting mixture refluxed for 5 h. After cooling the solvent was removed in vacuo and the residue was purified by flash chromatography. Elution with light petroleum-ethyl acetate (80:20) furnished 27 in 78% yield: mp 104.4-105.6 °C; 1H NMR (400 MHz) δ 5.57 (m, 1H), 5.39 (m, 1H), 3.6 (m, 1H), 5.38-5.40 (m, 1H), 5.56-5.58 (m, 1H); 13C NMR (100 MHz) 11.8, 12.6, 16.2, 18.6, 20.0, 21.0, 21.4, 22.9, 28.2, 30.0, 31.8, 32.2, 36.9, 38.3, 39.0, 40.7, 42.9, 46.1, 49.0, 52.7, 54.4, 55.5, 70.3, 116.4, 119.4, 139.91, 140.9, 215.1; Anal. Calcd for C28H44O2: C, 81.50%; H, 10.75%. Anal. Found: C, 81.73%; H, 10.71%.

### Example 15 (not according to the invention)

3β-Hydroxyergost-5,7-diene-22-one (28). The derivative 49 was treated as reported for 48 to furnish 28 in 58% yield: mp 118.2-122.6 °C. 1H NMR (400 MHz) δ 0.65 (s, 3H), 3.63-3.68 (m, 1H), 5.39-5.40 (m, 1H), 5.57-5.59 (m, 1H); 13C NMR (100 MHz) 11.94, 15.88, 16.23, 16.70, 18.17, 20.95, 23.19, 27.34, 31.82, 31.92, 33.61, 36.93, 38.26, 38.95, 40.65, 43.00, 46.07, 46.66, 49.98, 51.81, 53.66, 70.30, 116.56, 119.42, 139.97, 140.47, 214.79; Anal. Calcd for C28H44O2: C, 81.50%; H, 10.75%. Anal. Found: C, 81.70%; H, 10.78%.

### Example 16

(22R,23R)-22,23-Epoxy-3α,5α-cyclo-6β-methoxystigmastane (30) and (22S,23SR)-22,23-Epoxy-3α,5α-cyclo-6β-methoxystigmastane (31). NaHCO3 (7.34 g, 87 mmol,) and 77% m-CPBA (3.54 g, 16.8 mmol) were added to the solution of (22E)-3α,5α-cyclo-6β-methoxystigmast-22-ene34 (29) (3.0 g, 7.0 mmol) in CHCl3 (60 mL) and the resulting mixture was refluxed for 2 h. After cooling the reaction mixture was washed with 10% Na2S2O3 solution (3x50 mL), water (50 mL), and then dried over Na2SO4. The solvent was removed in vacuo and the residue submitted to mpc. Elution by light petroleum-ethyl acetate (95:5) afforded pure samples of 30 and 31 in 30% and 18% yields, respectively. Their spectral data were in agreement with those previously reported.48

### Example 17 (not according to the invention)

23R)-3β-Acetoxystigmast-5-ene-23-ol (36) and (22R)-3β-Acetoxystigmast-5-ene-22-ol (37). LiAlH4 (0.22 g, 5.94 mmol) was portion wise added to the solution of the epoxide 31 (0.24 g, 0.54 mmol) in anhydrous THF (15 mL). The resulting mixture was refluxed for 36 h under an argon atmosphere. After cooling, first EtOAc and then water were carefully added. The organic phase was separated and the water phase extracted with EtOAc (3x15 mL). The combined organic phases were washed with brine (20 mL) and then dried over Na2SO4. After filtration, the solvent was evaporated in vacuo to give the mixture of 34 + 35, which was dissolved in glacial acetic acid (10 mL) and the resulting solution refluxed for 3 h. After cooling, the solvent was removed in vacuo to give a residue, which was submitted to mpc. Elution by light petroleum-ethyl acetate (80:20) afforded pure sample of (23R)-3β-acetoxystigmast-5-ene-23-ol (36): 36% yield; mp 132.1-132.6 °C; 1H NMR (400 MHz) δ 0.70 (s, 3H), 2.01 (s, 3H), 2.30 (d, 2H, J = 7.56 Hz), 3.67-3.71 (m, 1H), 4.58 (m, 1H), 5.36 (d, 1H, J = 4.25 Hz); 13C NMR (100 MHz) 11.86, 14.56, 18.53, 18.82, 19.03, 19.22, 20.93, 21.37 (2C), 24.17, 27.69 (2C), 28.45, 31.72, 31.79, 32.70, 36.47, 36.89, 38.02, 39.68, 40.93, 42.38, 49.88, 52.33, 56.70, 56.76, 70.04, 73.88, 122.53, 139.51, 170.48. Further elution with the same eluent afforded (22R)-3β-acetoxystigmast-5-ene-22-ol (37): 21% yield; mp 123.9-125.2 °C; 1H NMR (400 MHz) δ 0.70 (s, 3H), 2.03 (s, 3H), 2.31 (d, 2H, J = 7.11 Hz), 3.71 (d, 1H, J = 10.12 Hz), 4.60 (m, 1H), 5.37 (s, 1H); 13C NMR (100 MHz) 11.77, 11.81, 12.26, 17.53, 19.26, 20.52, 20.96, 21.41, 23.50, 24.36, 27.39, 27.69, 28.62, 29.77, 31.80 (2C), 36.50, 36.93, 38.04, 39.61, 41.30, 42.45, 42.57, 49.97, 52.90, 56.18, 71.19, 73.88, 122.51, 139.59, 170.54.

### Example 18

3β-Acetoxy-5α,8α-(3,5-dioxo-4-phenyl-1,2,4-triazolidino)-22,23-epoxyergost-6-ene (41a). 77% m-CPBA (0.42 g, 1.87 mmol) was added to the solution of ergosterol acetate adduct 4036 (1.0 g, 1.63 mmol) in CH2Cl2 (10 mL) and the resulting mixture was stirred at room temperature for 5 h. Then, the reaction mixture was filtered and the solution washed with 5% NaHCO3 solution (2x10 mL) and brine (10 mL). The organic phase was dried over Na2SO4, filtered and the solvent was removed in vacuo to give a residue which was submitted to flash chromatography. Elution by light petroleum-ethyl acetate (90:10) afforded the desired compound 41a in 80% yield: mp: 138.1-144.1 °C; 1H NMR (400 MHz) δ 2.25-2.75 (m, 4H), 3.15-3.25 (m, 1H), 5.40 (m, 1H), 6.25 (m, 1H), 6.40 (m, 1H), 7.25-7.50 (m, 5H). 13C NMR (100 MHz) δ: 12.3, 12.8, 13.0, 13.4, 17.1, 17.3, 18.5, 19.2, 20.1, 20.3, 21.1, 22.2, 25.7, 30.7, 30.9, 33.5, 37.8, 39.3, 40.9, 42.3, 44.0, 48.8, 52.6, 54.9, 60.1, 62.8, 63.9, 64.6, 64.7, 65.1, 70.2, 126.0, 127.6, 128.6, 128.9, 131.5, 135.0, 135.3, 146.4, 148.8, 148.9, 169.8.

### Example 19 (not according to the invention)

(22R,23R)-3β-Acetoxy-22,23-epoxyergosta-5,7-diene (42) and (22S,23S)-3β-Acetoxy-22,23-Epoxyergosta-5,7-diene (43). Anhydrous K2CO3 (0.13 g, 0.93 mmol) was added to a solution of epoxide 41a (0.59 g, 0.93 mmol) in anhydrous DMF (50 mL). The resulting mixture was refluxed for 6 h, then cooled to room temperature and neutral alumina was added. The resulting mixture was filtered, and treated with water to yield a precipitate, which was then filtered in vacuo washing with water. The solid was submitted to mpc. Elution with light petroleum-ethyl acetate (90:10) afforded pure samples of the desired compounds in 65% total yield; 42: mp 158.8-160.2 °C; 1H NMR (400 MHz) δ 0.62 (s, 3H), 2.05 (s, 3H), 2.46-2.48 (m, 2H), 2.60-2.62 (m, 1H), 4.71 (m, 1H), 5.40-5.41 (m, 1H), 5.57-5.58 (m, 1H). 13C NMR (100 MHz) 11.92, 13.70, 16.09, 16.29, 19.51, 20.43, 20.94, 21.43, 23.21, 26.83, 28.06, 31.10, 36.60, 37.04, 37.87, 39.00, 42.29, 43.20, 46.00, 53.94, 55.63, 60.37, 64.22, 72.74, 116.52, 120.19, 138.59, 141.05, 170.56; 43: mp 133.5-135.2 °C; 1H NMR (400 MHz) δ 0.61 (s, 3H), 2.05 (s, 3H), 2.37-2.52 (m, 3H), 2.69 (d, 1H, J = 7.70 Hz), 4.71 (m, 1H), 5.40 (bs, 1H), 5.57 (bs, 1H). 13C NMR (100 MHz) 11.88, 12.56, 16.13, 17.14, 18.57, 20.24, 20.92, 21.43, 23.32, 27.80, 28.05, 31.00, 36.61, 37.05, 37.86, 38.92, 39.89, 42.50, 43.22, 45.95, 53.28, 53.97, 63.07, 63.83, 72.72, 116.53, 120.08, 138.78, 140.94, 170.57.

### Example 20 (not according to the invention)

(23R)-3β-(Tetrahydro-2H-pyran-2-yloxy)ergost-5,7-diene-23-ol (44) and (22R)-3β-(Tetrahydro-2H-pyran-2-yloxy)ergost-5,7-diene-22-ol (47). LiAlH4 (1.87 g, 49 mmol) was portion wise added to the solution of the epoxide 41b (2.83 g, 4.2 mmol) in anhydrous THF (110 mL). The resulting mixture was refluxed for 36 h under an argon atmosphere. After cooling, first EtOAc and then water were carefully added. The organic phase was separated and the water phase extracted with EtOAc (3x25 mL). The combined organic phases were washed with brine (30 mL), dried over Na2SO4, filtered and the solvent removed in vacuo to give a residue which was submitted to mpc. Elution with light petroleum-ethyl acetate (95:5) gave a pure sample of 44 in 20.5% yield: mp 95.1-96.9 °C; 1H NMR (400 MHz) δ 0.66 (s, 3H), 3.49-3.51 (m, 1H), 3.62-3.74 (m, 2H), 3.93-3.95 (m, 1H), 4.74-4.77 (m, 1H), 5.39 (s, 1H), 5.57 (s, 1H); 13C NMR (100 MHz) 9.79, 11.82, 16.14, 18.40, 18.76, 19.78, 19.95, 20.97, 21.46, 22.94, 25.42, 28.20, 28.28, 29.50, 29.92, 31.13, 31.22, 33.05, 37.19, 37.34, 38.17, 38.46, 38.68, 39.17, 42.09, 42.95, 45.35, 46.12, 54.46, 56.45, 62.54, 62.77, 70.53, 74.55, 74.69, 96.60, 97.00, 116.31, 116.40, 119.34, 119.47, 139.89, 140.14, 140.78, 141.01. Further elution gave the inseparable mixture of (23S)-3β-tetrahydropyranyloxyergost-5,7-diene-23-ol (45) and (22S)-3β-tetrahydropyranyloxyergost-5,7-diene-22-ol (46) in 30% yield. Following elution afforded a pure sample of 47 in 21% yield: mp 180.2-181.5 °C; 1H NMR (400 MHz) δ 0.63 (s, 3H), 1.07 (d, 3H, J = 6.50 Hz), 3.47-3.50 (m, 1H), 3.61-3.65 (m, 2H), 3.76 (d, 1H, J = 10.66 Hz), 3.91-3.93 (m, 1H), 4.73-4.75 (m, 1H), 5.37 (s, 1H), 5.55 (s, 1H); 13C NMR (100 MHz) 10.54, 11.68, 11.76, 12.48, 15.57, 16.06, 16.18, 17.91, 19.84, 20.01, 20.67, 21.01, 21.74, 23.05, 23.15, 25.45, 27.40, 27.73, 28.23, 28.38, 29.57, 29.96, 31.17, 31.26, 34.59, 35.31, 35.73, 37.23, 37.37, 38.22, 38.50, 38.73, 39.12, 40.72, 42.97, 43.05, 43.20, 45.35, 46.11, 46.16, 52.75, 53.98, 54.34, 56.78, 62.61, 62.84, 71.61, 74.20, 74.60, 74.68, 96.67, 97.05, 116.33, 116.43, 116.52, 119.36, 119.48, 139.98, 140.07, 140.22, 140.31, 140.57, 140.81, 141.07.

### Example 21 (not according to the invention)

3β-(Tetrahydro-2H-pyran-2-yloxy)ergost-5,7-diene-23-one (48). A solution of DMSO (0.20 g, 2.51 mmol) in anhydrous CH2Cl2 (0.5 mL) was added to a solution of oxalyl chloride (0.17 g, 1.32 mmol) in anhydrous CH2Cl2 (1 mL), kept at -60 °C under an argon atmosphere. After the resulting mixture was stirred for 15 min at -60 °C, the solution of alcohol 44 (0.33 g, 0.66 mmol) in anhydrous CH2Cl2 (2 mL) was added. The mixture was stirred for 2 h at -55/60 °C before the addition of Et3N (0.51 g, 5.0 mmol). After the reaction mixture was allowed to reach room temperature, stirring was continued for 15 min, and then water (10 mL) was added. The reaction mixture was extracted with CH2Cl2 (3x5 mL), the combined organic layers were washed with brine (10 mL), dried over Na2SO4, filtered and the solvent removed in vacuo, to give a residue which was submitted to flash chromatography. Elution with light petroleum-ethyl acetate (90:10) furnished 48 in 65% yield: mp 135.9-136.3 °C; 1H NMR (400 MHz) δ 0.66 (s, 3H), 3.49 (m, 1H), 3.63 (m, 1H), 3.93 (m, 2H), 5.38 (s, 1H), 5.56 (s, 1H); 13C NMR (100 MHz) 11.82, 12.58, 16.23, 18.67, 20.07, 21.38, 22.98, 25.48, 28.20, 30.08, 31.30, 32.28, 37.28, 38.00, 39.09, 43.01, 46.16, 49.09, 52.80, 54.47, 55.66, 62.91, 74.70, 74.76, 97.13, 116.53, 119.45, 139.89, 140.91, 214.81.

### Biology.

T0901317, GW4064 and 9-cis-retinoic acid were purchased from Sigma. Rosiglitazone was purchased from Cayman Chemical (Ann Arbor, MI).

Cell Culture and Co-transfection Assays. Human embryonic Kidney 293 cells (American Type Culture Collection) were cultured in Dulbecco's Modified Eagle's medium containing 10% of fetal bovine serum at 37°C in humidified atmosphere of 5% CO2. HEK293 cells (4x104 cells per well) were transiently transfected in 48 well plate with the reporter plasmids pMH100X4-TK-luc (100 ng/well), Renilla (22 ng/well) together with 100 ng/well of pCMX-Gal4-RXR, pCMX-Gal4-PPAR-y, pCMX-Gal4-PXR, pFA-CMV-FXR pCMX-Gal4-LXR-α or pCMX-Gal4-LXR-β plasmids using X-tremeGENE 9 DNA Transfection Reagent (Roche). Six hours after transfection, the cells were treated with the appropriate compound for 24 hours. Luciferase activities were analyzed by luciferase Dual Reporter Assay Systems (Promega) according to the manufacturer's protocol. GAL4-LXRs, GAL4-PPAR-y, GAL4-RXR and TK-MHC100-luc plasmids were described in Villablanca et al.49 GAL4-PXR was a kind gift of Dr. Enrique Sainz (The Scripps Research Institute, La Jolla, USA). GAL4-FXR was a kind gift of Dr. Daniel Merk (Goethe-University Frankfurt am Main). The results obtained by luciferase assays and reported in Table 1 are from three to five independent experiments.

Quantitative Real-Time-PCR. U937 cell line was differentiated in foam macrophages with phorbol 12-myristate 13-acetate (PMA) 10 ng/ml (Sigma) for 72 hours at 37°C in 10 mm dish at the concentration of 3x106 cells in 10 ml RPMI 10% FBS. At day 3 nuclear receptor ligands were added for 6 hours. HepG2 cells were treated with the ligands as described by Quinet et al.22 Total RNA was purified by TRIZOL (Invitrogen, Carlsbad, CA, USA). Reverse transcription was performed incubating 2 µg of total RNA 1 hour at 42 °C with MLV-reverse transcriptase (Promega). Quantitative PCR was performed using Sybr Green Master Mix (Applied Biosystems) and real-time PCR (Viia 7 Real Time PCR System, Applied Biosystems). All PCR reactions were done in triplicate. The comparative Ct method was used to quantify transcripts that were normalized for human GAPDH. the following primer pairs were used:

| | |
|---|---|
| GAPDH-F | ACA TCA TCC CTG CCT CTA CTG |
| GAPDH-R | ACC ACC TGG TGC TCA GTG TA |
| ABCA1-F | CCA GGC CAG TAC GGA ATT C |
| ABCA1-R | CCT CGC CAA ACC AGT AGG A |
| SREBP-1c-F | GGC GGG CGC AGA TC |
| SREBP-1c-R | TTG TTG ATA AGC TGA AGC ATG TCT |
| MCP-1-F | AGA AGC TGT GAT CTT CAA GAC CAT T |
| MCP-1-R | TGC TTG TCC AGG TGG TCC AT |
| FAS-F | ACA GCG GGG AAT GGG TAC T |
| FAS-R | GAC TGG TAC AAC GAG CGG AT |
| SCD1-F | TTC AGA AAC ACA TGC TGA TCC TCA TAA TTC |
| SCD1-R | ATT AAG CAC CAC AGC ATA TCG CAA GAA AGT |
| TNFα-F | TCT TCT CGA ACC CCG AGT GA |
| TNFα-R | CCT CTG ATG GCA CCA CCA G |

Statistical analysis. Data are expressed as mean ± SEM and were analyzed for significance by ANOVA with Dunnet's multiple comparison tests. The analysis was performed with Prism software. Data in Table 1 are expressed as EC50 ± SD. In particular, the standard deviations were obtained by calculating the mean of the EC50 of each experiment (three to five independent experiments). The efficacy (%) of the compounds was calculated as the percentage of the compound effect, in terms of LXRα or β activation, versus 8 µM of 22R-HC ± SD. The analyses were performed with Prism software.

X-ray Analysis. A single crystal of compound 23 was submitted to X-ray data collection on an Oxford-Diffraction Xcalibur Sapphire 3 diffractometer with a graphite monochromated Mo-Kα radiation (λ = 0.71073 Å) at 293 K. The structure was solved by direct methods implemented in SHELXS program (version 2013/1).50 The refinement was carried out by full-matrix anisotropic least-squares on F2 for all reflections for non-H atoms by means of the SHELXL program (version 2013/4).50 Crystallographic data (excluding structure factors) of 23 have been deposited with the Cambridge Crystallographic Data Centre as supplementary publication no. CCDC 1526884. Copies of the data can be obtained, free of charge, on application to CCDC, 12 Union Road, Cambridge CB2 1EZ, UK (deposit@ccdc.cam.ac.uk).

### Pharmacological assays on murine Lewis lung carcinoma cells (LLC) in vitro and in vivo

We have performed a set of experiments to evaluate whether some LXR modulators, particularly PFM009 (13) and PFM018 (25) influence the growth of the murine Lewis lung carcinoma cells (LLC) *in vitro* and *in vivo.* The treatment of LLC tumor cells for 96 hours with 10 µM of the above-reported compounds was not able to alter LLC tumor growth *in vitro* (Figure 11). We tested by qPCR whether these compounds were able to induce the expression of the LXR target genes in LLC cells, and we observed that both PFM009 and PFM018 induced the expression of the LXR target gene Abca1 in LLC tumor cells (Figure 12).

Then, we tested whether these compounds could influence LLC tumor growth in vivo. To do that, C57BL/6 mice were challenged with 1×10⁶ LLC tumor cells subcutaneously. Six days later, tumor-bearing mice were treated with PFM009 or PFM018 (30 mg/Kg/day) for five consecutive days and then evaluated for tumor growth. In accordance with data obtained by qPCR experiments, we observed a significant control of LLC tumor growth when mice were treated with both PFM009 and PFM018 (Figure 13A and 13B). While not willing to be bound to any particular theory, these experiments seem to indicate that PFM009 and PFM018, which are LXRαβ and β selective agonists, respectively, control tumor growth possibly interfering with non-tumor microenvironmental cells, as these compounds do not block tumor cell proliferation *in vitro* (Figure 11).

### REFERENCES

(1) Poli, G.; Biasi, F.; Leonarduzzi, G. Redox biology oxysterols in the pathogenesis of major chronic diseases. Redox Biol. 2013, 1, 125-130.
(2) Björkhem, I.; Diczfalusy, U. Oxysterols: friends, foes, or just fellow passengers? Arterioscler. Thromb. Vasc. Biol. 2002, 734-743.
(3) Bovenga, F.; Sabbà, C.; Moschetta, A. Uncoupling nuclear receptor LXR and cholesterol metabolism in cancer. Cell Metab. 2015, 21, 517-526.
(4) Traversari, C.; Sozzani, S.; Steffensen, K. R.; Russo, V. LXR-dependent and - independent effects of oxysterols on immunity and tumor growth. Eur. J. Immunol. 2014, 44, 1896-1903.
(5) Venteclef, N.; Ferr6, P. Liver X receptor: from metabolism to cancer. Biochem. J. 2014, 459, e1-e3.
(6) De Boussac, H.; Alioui, A.; Viennois, E.; Dufour, J.; Trousson, A.; Vega, A.; Guy, L.; Volle, D. H.; Lobaccaro, J.-M. A.; Baron, S. Oxysterol receptors and their therapeutic applications in cancer conditions. Expert Opin. Ther. Targets 2013, 17, 1029-1038.
(7) Janowsky, Bethany A.; Willy, P. J.; Rama Devi, T.; Falck, J. R.; Mangelsdorf, D. J. An oxysterol signalling pathway mediated by the nuclear receptor LXR. Nature 1996, 383, 728-731.
(8) Janowski, B. A.; Grogan, M. J.; Jones, S. A.; Wisely, G. B.; Kliewer, S. A.; Corey, E. J.; Mangelsdorf, D. J. Structural requirements of ligands for the oxysterol liver X receptors LXR α and LXR β. Proc. Natl. Acad. Sci. U. S. A. 1999, 96, 266-271.
(9) Song, C.; Kokontis, J. M.; Hiipakka, R. A.; Liao, S. Ubiquitous receptor: a receptor that modulates gene activation by retinoic acid and thyroid hormone receptors. Proc. Natl. Acad. Sci. USA 1994, 91, 10809-10813.
(10) Shinar, D. M.; Endo, N.; Rutledge, S. J.; Vogel, R.; Rodan, G. A.; Schmidt, A. NER, a new member of the gene family encoding the human steroid hormone nuclear receptor. Gene 1994, 147, 273-276.
(11) Apfel, R.; Benbrook, D.; Lernhardt, E.; Ortiz, M. A.; Salbert, G.; Pfahl, A. M. A novel orphan receptor specific for a subset of thyroid hormone-responsive elements and its interaction with the retinoid / thyroid hormone receptor subfamily. Mol. Cell. Biol. 1994, 14, 7025-7035.
(12) Teboul, M.; Enmark, E.; Li, Q.; Wirkstroem, A. C.; Pelto-Huikko, M.; Gustafsson, J.-A. OR-1, a member of the nuclear receptor superfamily that interacts with the 9-cis-retinoic acid receptor. Proc. Natl. Acad. Sci. U. S. A. 1995, 92, 2096-2100.
(13) Willy, P. J.; Umesono, K.; Ong, E. S.; Evans, R. M.; Heyman, R. a; Mangelsdorf, D. J. LXR, a nuclear receptor that defines a distinct retinoid response pathway. Genes Dev. 1995, 9, 1033-1045.
(14) Spann, N. J.; Glass, C. K. Sterols and oxysterols in immune cell function. Nat. Immunol. 2013, 14, 893-900.
(15) Gabbi, C.; Warner, M.; Gustafsson, J.-Å. Action mechanisms of liver X receptors. Biochem. Biophys. Res. Commun. 2014, 446, 647-650.
(16) Dong, X.-Y.; Tang, S.-Q.; Chen, J.-D. Dual functions of Insig proteins in cholesterol homeostasis. Lipids Health Dis. 2012, 11, 173.
(17) Olkkonen, V. M.; Zhou, Y.; Yan, D.; Vihervaara, T. Oxysterol-binding proteins-emerging roles in cell regulation. Eur. J. Lipid Sci. Technol. 2012, 114, 634-643.
(18) Corman, A.; Deberardinis, A. M.; Hadden, M. K. Structure - activity relationships for side chain oxysterol agonists of the hedgehog signaling pathway. ACS Med. Chem. Lett. 2012, 3, 828-833.
(19) Tice, C. M.; Noto, P. B.; Fan, K. Y.; Zhuang, L.; Lala, D. S.; Singh, S. B. The medicinal chemistry of liver X receptor (LXR) modulators. J. Med. Chem. 2014, 57, 7182-7205.
(20) Loren, J.; Huang, Z.; Laffitte, B. a; Molteni, V. Liver X Receptor Modulators: A review of recently patented compounds (2009 - 2012). Expert Opin. Ther. Pat. 2013, 23, 1317-1335.
(21) Spencer, T. a.; Li, D.; Russel, J. S.; Collins, J. L.; Bledsoe, R. K.; Consler, T. G.; Moore, L. B.; Galardi, C. M.; McKee, D. D.; Moore, J. T.; Watson, M. a.; Parks, D. J.; Lambert, M. H.; Willson, T. M. Pharmacophore analysis of the nuclear oxysterol receptor LXRα. J. Med. Chem. 2001, 44, 886-897.
(22) Quinet, E. M.; Savio, D. a; Halpern, A. R.; Chen, L.; Miller, C. P.; Nambi, P. Gene-selective modulation by a synthetic oxysterol ligand of the liver X receptor. J. Lipid Res. 2004, 45, 1929-1942.
(23) Berrodin, T. J.; Shen, Q.; Quinet, E. M.; Yudt, M. R.; Freedman, L. P. Identification of 5α, 6α-epoxycholesterol as a novel modulator of liver X receptor activity. Mol. Pharmacol. 2010, 78, 1046-1058.
(24) Peng, D.; Hiipakka, R.; Dai, Q.; Guo, J.; Reardon, C.; Getz, G. S.; Liao, S. Antiatherosclerotic effects of a novel synthetic tissue-selective steroidal liver X receptor agonist in low-density lipoprotein receptor-deficient mice. J. Pharmacol. Exp. Ther. 2008, 327, 332-342.
(25) Peng, D.; Hiipakka, R.; Xie, J. T.; Dai, Q.; Kokontis, J. M.; Reardon, C.; Getz, G. S.; Liao, S. A novel potent synthetic steroidal liver X receptor agonist lowers plasma cholesterol and triglycerides and reduces atherosclerosis in LDLR -/- mice. Br. J. Pharmacol. 2011, 162, 1792-1804.
(26) Li, L.; Liu, J.; Zhu, L.; Cutler, S.; Hasegawa, H.; Shan, B.; Medina, J. C. Discovery and optimization of a novel series of liver X receptor-α Agonists. Bioorg. Med. Chem. Lett. 2006, 16, 1638-1642.
(27) Jones, P. J. H. Cholesterol-lowering effect of plant sterols. Curr. Atheroscler. Rep. 1999, 1, 230-235.
(28) Plat, J.; Mensink, R. P. Increased intestinal ABCA1 expression contributes to the decrease in cholesterol absorption after plant stanol consumption. FASEB J. 2002, 16, 1248-1253.
(29) Kaneko, E.; Matsuda, M.; Yamada, Y.; Tachibana, Y.; Shimomura, I.; Makishima, M. Induction of intestinal ATP-binding cassette transporters by a phytosterol-derived liver X receptor agonist. J. Biol. Chem. 2003, 278, 36091-36098.
(30) Chisholm, J. W.; Hong, J.; Mills, S. A.; Lawn, R. M. The LXR Ligand T0901317 induces severe lipogenesis in the db/db diabetic mouse. J. Lipid Res. 2003, 44, 2039-2048.
(31) Premalatha, R.; Srikumar, K.; Vijayalaksmi, D.; Kumar, G. N.; Mathur, P. P. 28-Homobrassinolide: a novel oxysterol transactivating LXR gene expression. Mol. Biol. Rep. 2014, 41, 7447-7461.
(32) Chen, Z.; Liu, J.; Fu, Z.; Ye, C.; Zhang, R.; Song, Y.; Zhang, Y.; Li, H.; Ying, H.; Liu, H. 24(S)-Saringosterol from edible marine seaweed Sargassum Fusiforme is a novel selective LXRβ agonist. J. Agric. Food Chem. 2014, 62, 6130-6137.
(33) Yang, C.; Yu, L.; Li, W.; Xu, F.; Cohen, J. C.; Hobbs, H. H. Disruption of cholesterol homeostasis by plant sterols. J. Clin. Invest. 2004, 114, 813-822.
(34) Foley, D.; O'Callaghan, Y.; O'Brien, N. M.; McCarthy, F. O.; Maguire, A. R. Synthesis and characterization of stigmasterol oxidation products. J. Agric. Food Chem. 2010, 58, 1165-1173.
(35) Misharin, A. Y.; Mehtiev, A. R.; Morozevich, G. E.; Tkachev, Y. V; Timofeev, V. P. Synthesis and cytotoxicity evaluation of 22,23-oxygenated stigmastane derivatives. Bioorg. Med. Chem. 2008, 16, 1460-1473.
(36) Crump, D. R.; Williams, D. H.; Pelc, B. (22S)-Hydroxyvitamin D4. J. C. S. Perkin I 1973, 2731-2733.
(37) Tada, M.; Oikawa, A. Synthesis of 22,23-epoxyvitamin D2 (22,23-Epoxyergocalciferol). J. C. S. Perkin I 1979, 1858-1861.
(38) Miyashita, N.; Yoshikoshi, A.; Grieco, P. A. Pyridinium p-toluenesulfonate. A mild and efficient catalyst for the tetrahydropyranylation of alcohols. J. Org. Chem. 1977, 42, 3772-3774.
(39) Zhang, R.; He, H. P.; Di, Y. T.; Li, S. L.; Zuo, G. Y.; Zhang, Y.; Hao, X. J. Chemical constituents from Aphanamixis Grandifolia. Chem. Nat. Compd. 2013, 49, 100-104.
(40) Wei, X.; Shu, P.; Liu, T.; Xiang, M.; Zhang, J.; Xue, Y.; Luo, Z.; Yao, G.; Zhang, Y. Steroids and phenylpropanoids with immunomodulatory activities from the stem barks of Cinnamomum Wilsonii. Chinese J. Org. Chem. 2013, 33, 1273.
(41) Brynjolffssen, J.; Hands, D.; Midgley, J. M.; Whalley, W. B. Unsaturated steroids. Part I. Synthesis of 22,23-dihydroergosterol. J. C. S. Perkin I 1976, 826-828.
(42) Barton, D. H. R.; Poyster, J. P.; Sammes, P. G.; Hursthouse, M. B.; Neidle, S. Stereospecific and regiospecific addition to an isolated, acyclic (steroidal) olefinic bond. Chem. Commun. 1971, 715-716.
(43) Hong, C.; Tontonoz, P. Liver X receptors in lipid metabolism: opportunities for drug discovery. Nat. Rev. Drug Discovery 2014, 13, 433-444.
(44) Repa, J. J.; Turley, S. D.; Lobaccaro, J. a; Medina, J.; Li, L.; Lustig, K.; Shan, B.; Heyman, R. a; Dietschy, J. M.; Mangelsdorf, D. J. Regulation of absorption and ABC1-mediated efflux of cholesterol by RXR heterodimers. Science 2000, 289, 1524-1529.
(45) Venkateswaran, A.; Laffitte, B. A.; Joseph, S. B.; Mak, P. A.; Wilpitz, D. C.; Edwards, P. A.; Tontonoz, P. Control of cellular cholesterol efflux by the nuclear oxysterol receptor LXRα. Proc. Natl. Acad. Sci. U. S. A. 2000, 97, 12097-12102.
(46) Glass, C. K.; Saijo, K. Nuclear receptor transrepression pathways that regulate inflammation in macrophages and T cells. Nat. Rev. Immunol. 2010, 10, 365-376.
(47) Ito, A.; Hong, C.; Rong, X.; Zhu, X.; Tarling, E. J.; Hedde, P. N.; Gratton, E.; Parks, J.; Tontonoz, P. LXRs link metabolism to inflammation through Abca1-dependent regulation of membrane composition and TLR signaling. Elife 2015, 4, 1-23.
(48) González Sierra, M.; Bustos, D. A.; Zudenigo, M. E.; Rúveda, E. A. Configurational assignment of epimeric 22,23-epoxides of steroids by Carbon-13 NMR Spectroscopy. Tetrahedron 1986, 42, 755-758.
(49) Villablanca, R. J.; Raccosta, L.; Zhou, D.; Fontana, R.; Maggioni, D.; Negro, A.; Sanvito, F.; Ponzoni, M.; Valentinis, B.; Bregni, M.; Prinetti, A.; Steffensen, K. R.; Sonnino, S.; Gustafsson, J.-A.; Doglioni, C.; Bordignon, C.; Traversari, C.; Russo, V. Tumor-mediated liver X receptor-α activation inhibits CC chemokine receptor-7 expression on dendritic cells and dampens antitumor rsponses. Nat. Med. 2010, 16, 98-105.
(50) Sheldrick, G. M. A short history of SHELX. Acta Cryst. 2008, A64, 112-122.

## Claims

1. A compound of formula (I) 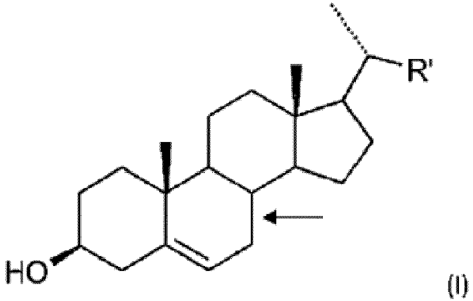 wherein
the bond indicated by the arrow is a single or a double bond; and
R' is the following group: 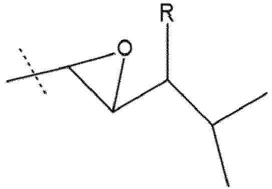 wherein
- the dotted line indicates the bond by which said group is linked to the remaining part of the molecule;
- R is a linear or branched, saturated C₁-C₆-alkyl; and
as well as all their possible stereoisomers for use in therapy.

2. The compound of formula (I) for use according to claim 1, **characterized in that** it is selected from the following compounds:

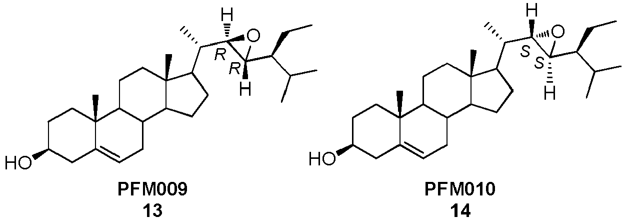

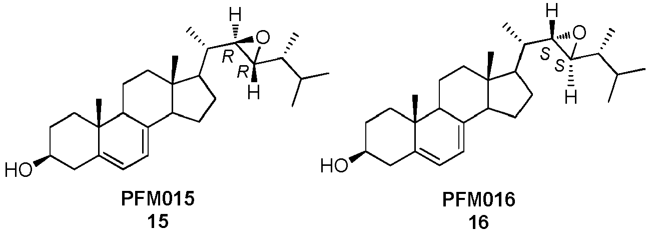

3. A compound of formula (I) for its use according to claim 1 or 2, selected from the compounds having the following formulas:

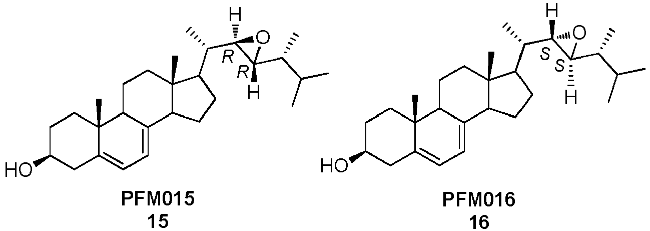

4. A compound of formula (I) for its use according to anyone of claims 1 to 3, in the prevention and/or treatment of diseases linked to LXR as well as in the prevention and/or treatment of cancer, inflammatory and/or immunological diseases, melanoma, prostate cancer and skin carcinoma, chronic inflammatory diseases, metabolic diseases and autoimmune diseases.

5. A pharmaceutical composition comprising a compound as defined in anyone of claims 1 to 3 with at least a pharmaceutically acceptable carrier, for its use in the prevention and/or treatment of diseases linked to LXR as well as in the prevention and/or treatment of cancer, inflammatory and/or immunological diseases, melanoma, prostate cancer and skin carcinoma, chronic inflammatory diseases, metabolic diseases and autoimmune diseases.

6. The composition for use according to claim 5, which comprises one or more additional active ingredients.

7. A compound of formula (II) 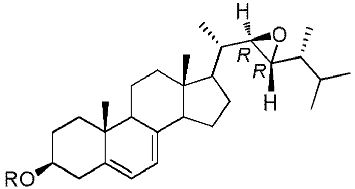 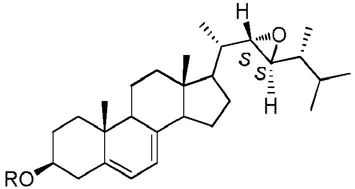 wherein R is an acetyl group.

## Patentansprüche

1. Eine Verbindung der Formel (I) 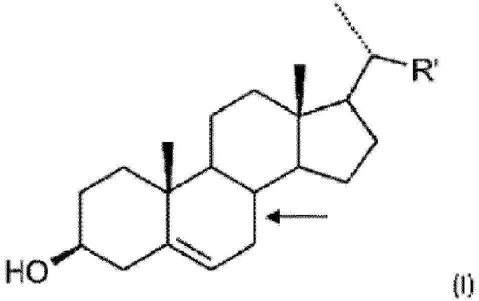 wobei
die durch den Pfeil gekennzeichnete Bindung eine Einfach- oder Doppelbindung ist; und
R' die folgende Gruppe ist: 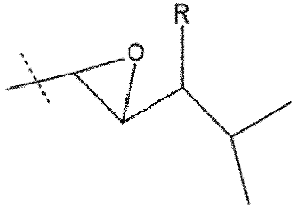
wobei
- die gestrichelte Linie die Bindung **kennzeichnet, durch** die diese Gruppe mit dem verbleibenden Teil des Moleküls verbunden ist;
- R ein lineares oder verzweigtes, gesättigtes C₁-C₆-Alkyl ist; und
sowie alle dessen möglichen Stereoisomere
zur Verwendung in der Therapie.

2. Die Verbindung der Formel (I) zu deren Verwendung gemäß Anspruch 1, **dadurch gekennzeichnet, dass** sie aus den folgenden Verbindungen ausgewählt ist:

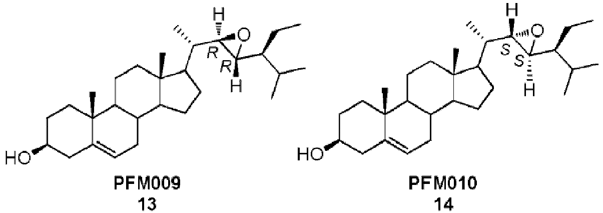

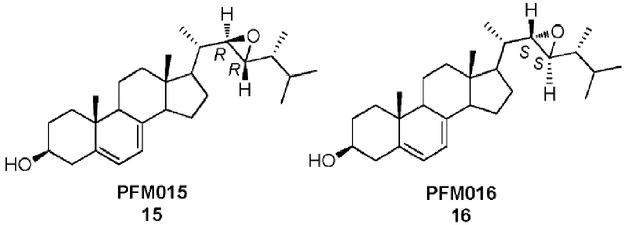

3. Eine Verbindung der Formel (I) zu deren Verwendung gemäß einem der Ansprüche 1 oder 2, wobei sie aus den Verbindungen mit den folgenden Formeln ausgewählt ist:

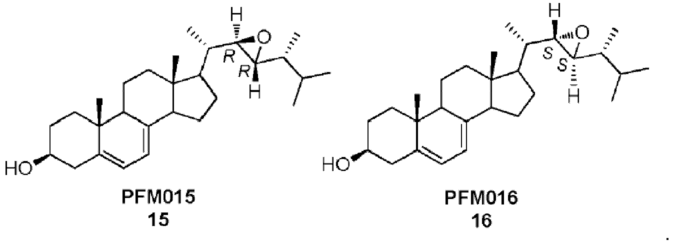

4. Eine Verbindung der Formel (I) zu deren Verwendung gemäß einem der Ansprüche 1 bis 3 bei der Vorbeugung und/oder Behandlung von mit LXR verbundenen Krankheiten sowie bei der Vorbeugung und/oder Behandlung von Krebs, entzündlichen und/oder immunologischen Erkrankungen, Melanomen, Prostatakrebs und Hautkrebs, chronisch entzündlichen Erkrankungen, Stoffwechselerkrankungen und Autoimmunerkrankungen.

5. Eine pharmazeutische Zusammensetzung, umfassend eine Verbindung gemäß einem der Ansprüche 1 bis 3 mit mindestens einem pharmazeutisch verträglichen Träger zu deren Verwendung bei der Vorbeugung und/oder Behandlung von mit LXR verbundenen Krankheiten sowie bei der Vorbeugung und/oder Behandlung von Krebs, entzündlichen und/oder immunologischen Erkrankungen, Melanomen, Prostatakrebs und Hautkrebs, chronisch entzündlichen Erkrankungen, Stoffwechselerkrankungen und Autoimmunerkrankungen.

6. Die Zusammensetzung nach Anspruch 5, die einen oder mehrere zusätzliche Wirkstoffe umfasst.

7. Eine Verbindung der Formel (II) 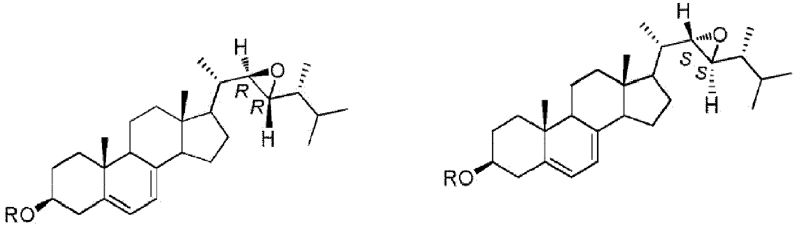 wobei R eine Acetylgruppe ist.

## Revendications

1. Composé de formule (I) 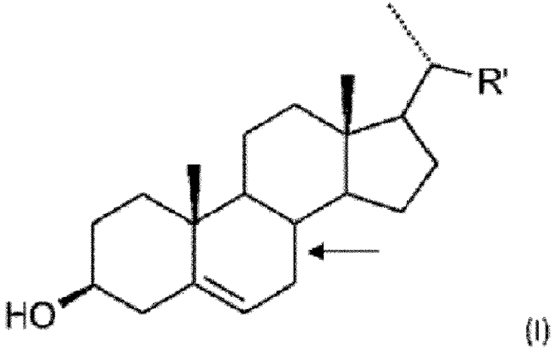 dans lequel
la liaison indiquée par la flèche est une liaison simple ou double ; et
R' représente le groupe suivant : 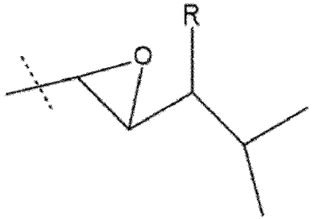
dans lequel
- la ligne pointillée indique la liaison par laquelle ledit groupe est lié à la partie restante de la molécule ;
- R représente un alkyle saturé en C₁-C₆, linéaire ou ramifié ; et
ainsi que tous leurs stéréoisomères possibles
pour une utilisation en thérapie.

2. Composé de formule (I) à utiliser selon la revendication 1, **caractérisé en ce qu'**il est choisi parmi les composés suivants :

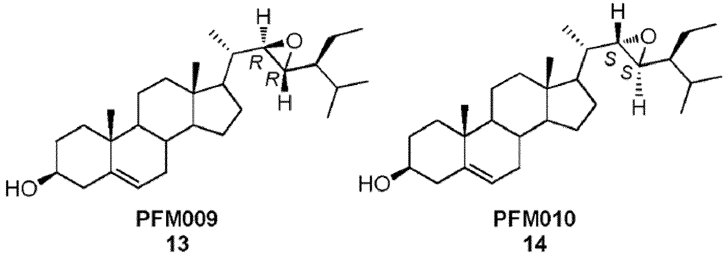

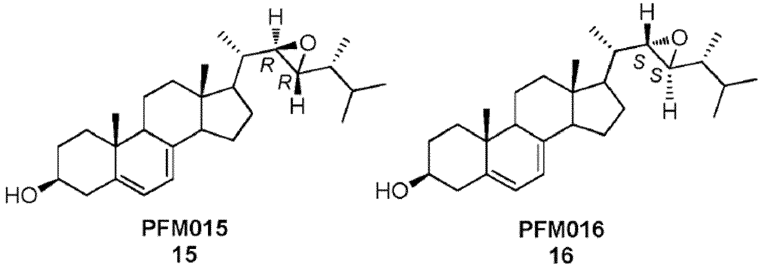

3. Composé de formule (I) pour son utilisation selon la revendication 1 ou 2, choisi parmi les composés ayant les formules suivantes :

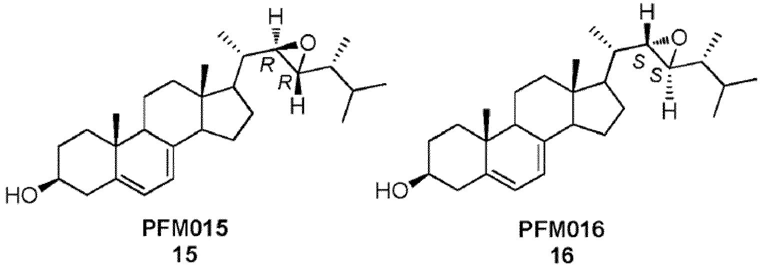

4. Composé de formule (I) pour son utilisation selon l'une quelconque des revendications 1 à 3, dans la prévention et/ou le traitement des maladies liées au LXR ainsi que dans la prévention et/ou le traitement du cancer, des maladies inflammatoires et/ou immunologiques, du mélanome, du cancer de la prostate et du carcinome cutané, des maladies inflammatoires chroniques, des maladies métaboliques et des maladies auto-immunes.

5. Composition pharmaceutique comprenant un composé tel que défini dans l'une quelconque des revendications 1 à 3 avec au moins un support pharmaceutiquement acceptable, pour son utilisation dans la prévention et/ou le traitement des maladies liées au LXR ainsi que dans la prévention et/ou le traitement du cancer, des maladies inflammatoires et/ou immunologiques, du mélanome, du cancer de la prostate et du carcinome de la peau, des maladies inflammatoires chroniques, des maladies métaboliques et des maladies auto-immunes.

6. Composition à utiliser selon la revendication 5, qui comprend un ou plusieurs ingrédients actifs supplémentaires.

7. Composé de formule (II) 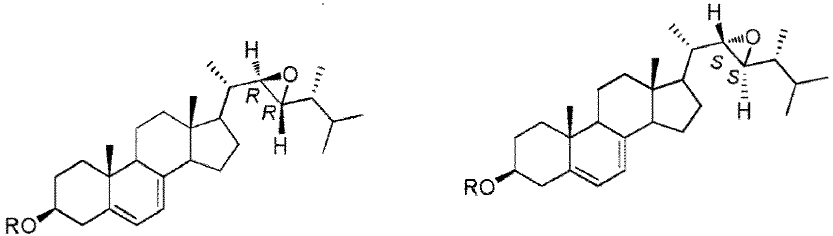 dans lequel R représente un groupe acétyle.
